# EUROPEAN PATENT SPECIFICATION

(11) **EP 4 456 880 B1**
(45) Date of publication and mention of the grant of the patent: **18.03.2026**
(21) Application number: 21851701.9
(22) Date of filing: 29.12.2021
(51) Int. Cl.: A61K 9/16, A61K 9/00, A61K 9/10, A61K 31/445, A61P 37/08, A61K 9/20

(54) **COMPOSITIONS CONTAINING FEXOFENADINE**
ZUSAMMENSETZUNGEN MIT FEXOFENADIN
COMPOSITIONS CONTENANT DE LA FEXOFÉNADINE

(43) Date of publication of application: 06.11.2024
(73) Proprietor: Opella Healthcare Group SAS, 92200 NEUILLY SUR SEINE (FR)
(72) Inventor: CAVALARI, Daniela Maldonado, 60200 Compiegne (FR); LIBOUREAU, Maxime, 60200 Compiegne (FR); ZAGHLOUL, Chakib, 60200 Compiegne (FR)
(74) Representative: Atout PI Laplace
(86) International application number: PCT/IB2021/000916
(87) International publication number: WO 2023/126637

(56) References cited:
- WO-A2-03/041683
- WO-A2-2011/151733
- CN-A- 105 997 910
- CN-B- 102 512 389
- KR-A- 20160 025 075

## Description

### TECHNICAL FIELD

The disclosure relates to compositions containing fexofenadine, processes for preparing the compositions.

### BACKGROUND

Allergies are a bothersome part of many individuals' lives, often affecting the quality of life of adults and children. Often, allergies cannot be escaped since they can be triggered by both indoor and outdoor allergens. Finding treatments for allergies can be difficult and often depend on the type of allergy and age of the patient.

A variety of allergy medications are known in the art and include allergy medications containing fexofenadine hydrochloride. Fexofenadine hydrochloride refers to 4-[1-hydroxy-4-[4-(hydroxydiphenylmethyl)-1-piperidinyl]-butyl]-α,α-dimethyl benzeneacetic acid hydrochloride. It has the below structure and is available in products such as Goodsense^{®} Aller-ease, Aller-Fex^{™}, Wal-Fex^{®} Allergy, Allegra^{®}, Allegra^{®} Allergy 12 Hour, Allegra^{®} Allergy 24 Hour, Children's Allegra^{®} Allergy, and Mucinex Allergy, Allegra^{®} Allergy, and Children's Wal-Fex^{®}.

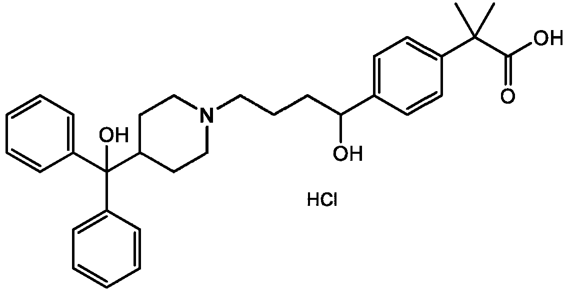

Fexofenadine hydrochloride has a bitter taste that can be unpalatable. Fexofenadine hydrochloride solid drug products available in the art are coated with a flavoring agent that masks its bitter taste. Alternatively, fexofenadine hydrochloride is mixed with a flavoring agent, but the flavoring agent cannot entirely mask the aversive, bitter taste.

In addition to having a bitter taste, routes to fexofenadine hydrochloride formulations are plagued with difficulties. For example, when fexofenadine hydrochloride formulations are dried, a significant amount of fexofenadine hydrochloride is lost, thereby reducing the yield of the final product. Further, since the fexofenadine hydrochloride particles are so small, there is the potential that the small particles may ignite during the drying process, thereby reducing the yield of the final product and possibly resulting in unsafe working conditions. Patent document WO 2011/151733 A2 discloses spray dried formulations comprising fexofenadine HCl.

What are needed are more efficient, sustainable, and safer methods for preparing drug products containing fexofenadine.

### SUMMARY

In certain embodiments, the disclosure provides processes of preparing a spray-dried formulation comprising fexofenadine zwitterion dihydrate comprising spray-drying (i) a fexofenadine zwitterion dihydrate composition; and (ii) an inert matrix.

In other embodiments, the disclosure provides products prepared according to the processes described herein.

In further embodiments, the disclosure provides spray-dried formulations comprising fexofenadine zwitterion dihydrate and an inert matrix.

In yet other embodiments, the disclosure provides spray-dried formulations, comprising fexofenadine zwitterion dihydrate, a wetting agent, an alkalizing agent, a buffer, a sweetener, a binder, a diluent, a disintegrant, a flavoring agent, and a lubricant.

In still further embodiments, the disclosure provides oral solid dosage forms comprising the spray-dried formulation described herein.

Although not claimed, the disclosure provides methods of relieving symptoms due to an allergy in a patient in need thereof, comprising administering a therapeutically effective amount of the spray-dried formulation or oral solid dosage form described herein to the patient.

Although not claimed, the disclosure provides methods of relieving symptoms due to an upper respiratory allergy in a patient in need thereof, comprising administering a therapeutically effective amount of the spray-dried formulation or the oral solid dosage form described herein to the patient.

Other aspects and embodiments of the invention will be readily apparent from the following detailed description of the invention.

### BRIEF DESCRIPTION OF THE DRAWINGS

The present application is further understood when read in conjunction with the appended drawings. For the purpose of illustrating the subject matter, there are shown in the drawings exemplary embodiments of the subject matter; however, the presently disclosed subject matter is not limited to the specific compositions, methods, and processes disclosed. In addition, the drawings are not necessarily drawn to scale.
Fig. 1 is a bar graph comparing the bitterness of fexofenadine formulations.
Fig. 2 is a differential scanning calorimetry (DSC) thermogram for fexofenadine hydrochloride.
Fig. 3 is a DSC thermogram for fexofenadine zwitterion dihydrate.

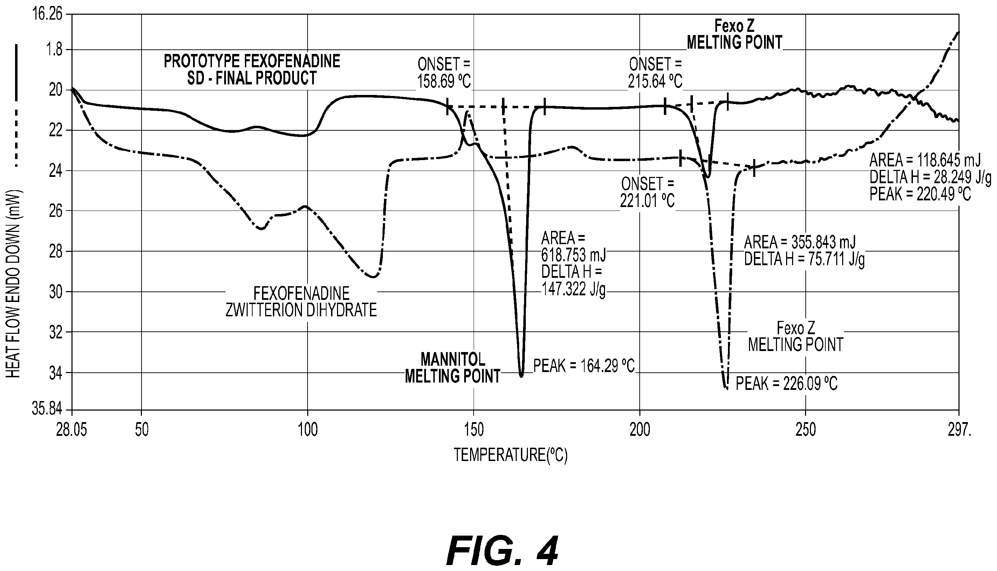

Fig. 4 is a DSC thermogram for fexofenadine zwitterion compared to a DSC thermograph of a spray-dried fexofenadine zwitterion dihydrate of the disclosure.

### DETAILED DESCRIPTION OF ILLUSTRATIVE EMBODIMENTS

As used herein, the singular forms "a", "an," and "the" include the plural reference. Reference to a particular numerical value includes at least that particular value unless the context clearly indicates otherwise. Thus, for example, reference to "a substance" is a reference to at least one of such substances and equivalents thereof known to those skilled in the art, and so forth.

When a value is expressed as an approximation by use of "about," it will be understood that the particular value forms another embodiment. In general, use of "about" indicates approximations that can vary depending on the desired properties sought to be obtained by the disclosed subject matter and is to be interpreted in the specific context in which it is used. The person skilled in the art will be able to interpret this as a matter of routine. In some instances, the number of significant figures used for a particular value may be one non-limiting method of determining the extent of the word "about." In other cases, the gradations used in a series of values may be used to determine the intended range available to the term "about" for each value. Where present, all ranges are inclusive and combinable. That is, references to values stated in ranges include every value within that range.

As used herein, while data is given with full disclosure of all significant figures, those of skilled in the art would understand that the data can also be understood when rounded to 1 or 2 decimal places.

When a list is presented, unless stated otherwise, it is to be understood that each individual element of that list and every combination of that list is to be interpreted as a separate embodiment. For example, a list of embodiments presented as "A, B, or C" is to be interpreted as including the embodiments "A," "B," "C," "A or B," "A or C," "B or C," or "A, B, or C."

It is to be appreciated that certain features of the invention which are, for clarity, described herein in the context of separate embodiments, may also be provided in combination in a single embodiment. That is, unless obviously incompatible or excluded, each individual embodiment is deemed to be combinable with any other embodiment(s) and such a combination is considered to be another embodiment. Conversely, various features of the invention that are, for brevity, described in the context of a single embodiment, may also be provided separately or in any sub-combination. It is further noted that the claims may be drafted to exclude any optional element. As such, this statement is intended to serve as antecedent basis for use of such exclusive terminology as "solely," "only" and the like in connection with the recitation of claim elements, or use of a "negative" limitation. Finally, while an embodiment may be described as part of a series of steps or part of a more general structure, each said step may also be considered an independent embodiment in itself.

The terms "patient" or "subject" as used herein are interchangeable and refer to a mammalian animal. In some embodiments, the patient or subject is a human. In other embodiments, the patient or subject is a veterinary or farm animal, a domestic animal or pet, or animal normally used for clinical research. In some embodiments, the patient is an adult, *i.e.,* 18 years of age or older. In other embodiments, the patient is a child, *i.e.,* under the age of 18.

The term "treating" includes ameliorating a disease or disorder (*i.e.,* arresting or reducing the development of the disease or at least one of the clinical symptoms thereof). In some embodiments, "treating" refers to ameliorating at least one physical parameter, which may not be discernible by the subject. In other embodiments, "treating" refers to modulating the disease or disorder, either physically, (e.g., stabilizing a discernible symptom), physiologically, (e.g., stabilizing a physical parameter), or both. In further embodiments, "treating" refers to delaying the onset of the disease or disorder.

### Processes

The present disclosure relates to compositions containing a form of fexofenadine that is not bitter tasting when administered orally. Such compositions may be used in a variety of oral dosage forms and are ready-to-use, *i.e.,* water is not required for the oral administration. In particular, the inventors discovered that fexofenadine zwitterion dihydrate could be formulated in compositions and oral dosage forms, whereby the fexofenadine zwitterion dihydrate does not convert to fexofenadine hydrochloride which has an undesirable bitter taste. By doing so, a bitterless drug product is provided for oral solid dosage forms, thereby promoting a satisfactory sensory experience (*e.g.*, taste and texture), without bitter aftertaste.

The terms "fexofenadine zwitterion" and "fexofenadine zwitterion dihydrate" are used interchangeably and refer to 4-[1-hydroxy-4-[4-(hydroxydiphenylmethyl)-1-piperidinyl]-butyl]-α,α-dimethyl benzeneacetic acid dihydrate. Fexofenadine zwitterion dihydrate has the following structure:

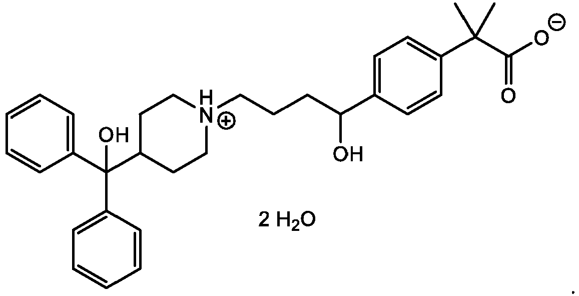

The processes used to prepare spray-dried fexofenadine zwitterion dihydrate formulations are advantageous for several reasons. For example, the processes can use conventional technologies and do not require special equipment, complicated reactions, expensive reagents, among others. Further, the processes to prepare fexofenadine zwitterion dihydrate are efficient, even for compositions containing high dosages of fexofenadine zwitterion dihydrate, which is contrary to what was reported in the art. In particular, fexofenadine formulations in the art were very bitter, particularly at high doses. The processes described herein provide formulations that are not bitter at any doses.

### A. Preparation of the Fexofenadine Zwitterion Dihydrate Composition

The fexofenadine zwitterion dihydrate for use in the spray-drying process is prepared as described herein. First, fexofenadine hydrochloride, water, and wetting agent are combined. In some embodiments, fexofenadine hydrochloride is combined with a solution comprising water and the wetting agent. The wetting agent is selected so as to facilitate the incorporation of fexofenadine hydrochloride in water and/or increase wettability. A number of wetting agent may be utilized and include, without limitation, a poloxamer, polysorbate, or polyoxyl hydrogenated castor oil.

The term "poloxamer" as used herein refers to nonionic triblock copolymers composed of consisting of a central hydrophobic block of polypropylene glycol flanked by two hydrophilic blocks of polyethylene glycol (PEG). Poloxamers are available in the art by the trade names Pluronic, Kolliphor, Lutrol, and Synperonic. In some embodiments, the wetting agent is a poloxamer such as poloxamer 188, poloxamer 237, poloxamer 338, poloxamer 407, or a combination thereof. The term "polysorbate" as used herein refers to a an oily liquids derived from ethoxylated sorbitan esterified with fatty acids. Polysorbates are available in the art as Kolliphor, Scattics, Alkest, Canarcel, and Tween products. In other embodiments, the wetting agent is a polysorbate such as polysorbate 20 (polyoxyethylene (20) sorbitan monolaurate), polysorbate 40 (polyoxyethylene (20) sorbitan monopalmitate), polysorbate 60 (polyoxyethylene (20) sorbitan monostearate), polysorbate 80 (polyoxyethylene (20) sorbitan monooleate), or SEPITRAP^{™} 80 (polysorbate 80 and magnesium aluminometasilicate). In further embodiments, the wetting agent is polysorbate 80 or SEPITRAP^{™} 80, or such as polysorbate 80, or such as SEPITRAP^{™} 80. In yet other embodiments, the wetting agent is a polyoxyl hydrogenated castor oil such as SEPITRAP^{™} 4000 (polyoxyl 40 hydrogenated castor oil and magnesium aluminosilicate). One or more than one wetting agent may be used. In some embodiments, one wetting agent is used. In other embodiments, two wetting agents are used. In further embodiments, three wetting agents are used.

Following the step with the wetting agent, the composition is combined with an alkalizing agent. The inventors discovered that use of alkalizing agent reduced the amounts of solids present in the compositions, thereby reducing viscosity. This permitted the composition to be spray-dried in later steps, while simultaneously maintaining the highest concentration of fexofenadine hydrochloride. Typically, the alkalizing agent is a strong base. The inventors found that by using the strong base, fexofenadine hydrochloride was converted to fexofenadine zwitterion dihydrate using less quantity of solids as compared to other routes, such as use of a buffer in this step. In some aspects, the alkalizing agent is potassium hydroxide, calcium hydroxide, sodium hydroxide, or barium hydroxide, among others. In other aspects, alkalizing agent is potassium hydroxide. In further aspects, the alkalizing agent is calcium hydroxide. In yet other aspects, the alkalizing agent is sodium hydroxide. In still further aspects, the alkalizing agent is barium hydroxide. One or more alkalizing agents may be used. In some embodiments, one alkalizing agent is used. In other embodiments, two alkalizing agents are used. In further embodiments, three alkalizing agents are used. The molar ratio of the alkalizing agent to fexofenadine hydrochloride is about 0.8:1 to about 1.2:1. In certain aspects, molar ratio of the alkalizing agent to fexofenadine hydrochloride is about 1:1.

The pH of the resulting composition can be adjusted *in situ* to about 5.8 to about 7 using one or more of a buffer. In some embodiments, the pH is adjusted to about 5.8, about 5.9, about 6, about 6.1, about 6.2, about 6.3, about 6.4, about 6.5, about 6.6, about 6.7, about 6.8, about 6.9, or about 7. In other embodiments, the pH is adjusted to about 5.8 to about 7, about 5.8 to about 6.8, about 5.8 to about 6.6, about 5.8 to about 6.4, about 5.8 to about 6.2, about 5.8 to about 6, 6 to about 7, about 6 to about 6.8, about 6 to about 6.6, about 6 to about 6.4, about 6 to about 6.2, about 6.2 to about 7, about 6.2 to about 6.8, about 6.2 to about 6.6, about 6.2 to about 6.4, about 6.4 to about 7, about 6.4 to about 6.8, about 6.4 to about 6.6, about 6.6 to about 7, about 6.6 to about 6.8, or about 6.8 to about 7. Examples of buffers include, without limitation, sodium phosphate dibasic 2H₂O, sodium phosphate monobasic, citric acid/sodium phosphate dibasic, sodium phosphate dibasic hydrate, succinic acid/sodium hydroxide, citric acid/sodium citrate, sodium citrate hydrate, potassium citrate, maleic acid/sodium hydroxide, fumaric acid/sodium hydroxide, sodium phosphate monobasic hydrate, potassium phosphate monobasic/sodium phosphate dibasic, sodium phosphate dibasic hydrate, potassium phosphate dibasic, potassium phosphate dibasic hydrate. In some embodiments, the buffer is sodium phosphate monobasic, sodium phosphate monobasic hydrate, potassium phosphate monobasic/sodium phosphate dibasic, sodium phosphate dibasic hydrate, potassium phosphate dibasic, or potassium phosphate dibasic hydrate. In other embodiments, the buffer is sodium phosphate monobasic, sodium phosphate monobasic hydrate/sodium phosphate dibasic, or sodium phosphate dibasic hydrate, or a combination thereof. In further embodiments, the buffer is phosphate dibasic and phosphate monobasic. One or more buffers may be used. In some embodiments, one buffer is used. In other embodiments, two buffers are used. In further embodiments, three buffers are used.

A sweetener can be combined with the resulting suspension containing the buffer. In some aspects, the sweetener is selected so as to provide a pleasant sensory experience to the patient. In certain aspects, the sweetener masks any salty taste that might result from NaCl that is generated when fexofenadine hydrochloride is converted to fexofenadine zwitterion dihydrate. The term "salty taste" as used herein refers to the taste that is recognized by taste buds of a person that corresponds to the same taste as salt, i.e., NaCl. The salty taste differs from the bitter taste that is provided by fexofenadine hydrochloride. The salty taste also is easier to mask than the bitter taste of fexofenadine hydrochloride. Examples of sweeteners include sucralose, aspartame, acesulfame potassium, saccharin, steviol glycoside, neotame, advantame, saccharin sodium, cyclamate sodium, or ammonium glycyrrhizate, or a combination thereof. In some aspects, the sweetener is sucralose. In other aspects, the sweetener is aspartame. In further aspects, the sweetener is acesulfame potassium. In yet other aspects, the sweetener is saccharin. In still further aspects, the sweetener is a steviol glycoside. In other aspects, the sweetener is neotame. In further aspects, the sweetener is advantame. In yet other aspects, the sweetener is saccharin sodium. In still further aspects, the sweetener is cyclamate sodium. In other aspects, the sweetener is ammonium glycyrrhizate. One or more sweeteners may be used. In some embodiments, one sweetener is used. In other embodiments, two sweeteners are used. In further embodiments, three sweeteners are used.

A binder can be combined with the resulting suspension containing the sweetener. The binder is selected so as to stabilize the formulation, e.g., steric stabilizing, and to avoid particle agglomeration. Examples of binders include, without limitation, hydroxypropylmethylcellulose (HPMC), hydroxypropylcellulose (HPC), polyvinylpyrrolidone (PVP), or carboxymethyl cellulose (CMC), hydroxyethylmethyl cellulose (HEMC), or hydroxyethylcellulose (HEC), or a combination thereof. In some aspects, the binder is HPMC. In other aspects, the binder is HPMC having a viscosity of about 4 to about 100,000 mPa.s. In further aspects, the binder is HPMC 4 mPa.s or HPMC 5 mPa.s. In yet other aspects, the binder is HPC. In still further aspects, the binder is PVP. In other aspects, the binder is CMC. In further aspects, the binder is HEMC. In yet other aspects, the binder is HEC. The composition may contain one or more binder. In some embodiments, the composition contains one binder. In other embodiments, the composition contains two binders. In further embodiments, the composition contains three binders.

The fexofenadine zwitterion dihydrate composition, including the alkalizing agent, buffer, sweetener, and binder, has a pH of about 5.8 to about 7. In some embodiments, the pH of the fexofenadine zwitterion dihydrate composition is about 5.8, about 5.9, about 6, about 6.1, about 6.2, about 6.3, about 6.4, about 6.5, about 6.6, about 6.7, about 6.8, about 6.9, or about 7. In other embodiments, the pH of the fexofenadine zwitterion dihydrate composition is about 5.8. In further embodiments, the pH of the fexofenadine zwitterion dihydrate composition is about 5.9. In yet other embodiments, the pH of the fexofenadine zwitterion dihydrate composition is about 6. In still further embodiments, the pH of the fexofenadine zwitterion dihydrate composition is about 6.1. In other embodiments, the pH of the fexofenadine zwitterion dihydrate composition is about 6.2. In further embodiments, the pH of the fexofenadine zwitterion dihydrate composition is about 6.3. In still other embodiments, the pH of the fexofenadine zwitterion dihydrate composition is about 6.4. In yet further embodiments, the pH of the fexofenadine zwitterion dihydrate composition is about 6.5. In still further embodiments, the pH of the fexofenadine zwitterion dihydrate composition is about 6.6. In other embodiments, the pH of the fexofenadine zwitterion dihydrate composition is about 6.7. In further embodiments, the pH of the fexofenadine zwitterion dihydrate composition is about 5.8 to about 7, about 5.8 to about 6.9, about 5.8 to about 6.8, about 5.8 to about 6.7, about 5.8 to about 6.6, about 5.8 to about 6.5, about 5.8 to about 6.4, about 5.8 to about 6.3, about 5.8 to about 6.2, about 5.8 to about 6.1, about 5.8 to about 6, about 5.8 to about 5.9, about 5.9 to about 7, about 5.9 to about 6.9, about 5.9 to about 6.8, about 5.9 to about 6.7, about 5.9 to about 6.7, about 5.9 to about 6.6, about 5.9 to about 6.5, about 5.9 to about 6.4, about 5.9 to about 6.3, about 5.9 to about 6.2, about 5.9 to about 6.1, about 5.9 to about 6, about 6 to about 7, about 6 to about 6.9, about 6 to about 6.8, about 6 to about 6.7, about 6 to about 6.6, about 6 to about 6.5, about 6 to about 6.4, about 6 to about 6.3, about 6 to about 6.2, about 6 to about 6.1, about 6.1 to about 7, about 6.1 to about 6.9, about 6.1, to about 6.8, about 6.1 to about 6.7, about 6.1 to about 6.6, about 6.1 to about 6.5, about 6.1 to about 6.4, about 6.1 to about 6.3, about 6.1 to about 6.2, about 6.2 to about 7, about 6.2 to about 6.9, about 6.2 to about 6.8, about 6.2 to about 6.7, about 6.2 to about 6.6, about 6.2 to about 6.5, about 6.2 to about 6.4, about 6.2 to about 6.3, about 6.3 to about 7, about 6.3 to about 6.9, about 6.3 to about 6.8, about 6.3 to about 6.7, about 6.3 to about 6.6, about 6.3 to about 6.5, about 6.3 to about 6.4, about 6.4 to about 7, about 6.4 to about 6.9, about 6.4 to about 6.8, about 6.4 to about 6.7, about 6.4 to about 6.6, about 6.4 to about 6.5, about 6.5 to about 6.9, about 6.5 to about 6.8, about 6.5 to about 6.7, about 6.5 to about 6.6, about 6.6 to about 7, about 6.6 to about 6.9, about 6.6 to about 6.8, about 6.6 to about 6.7, about 6.7 to about 7, about 6.7 to about 6.9, about 6.7 to about 6.8, about 6.8 to about 7, about 6.8 to about 6.9, or about 6.9 to about 7.

The processes for forming fexofenadine zwitterion dihydrate may be performed using equipment known in the art. In some embodiments, the fexofenadine zwitterion dihydrate composition is prepared using a rotor-stator homogenizer. During the preparation of the fexofenadine zwitterion dihydrate composition, the fexofenadine zwitterion dihydrate particles are suspended and air is incorporated into the composition. By doing so, the density of composition decreases and the volume of the composition increases.

### B. Preparation of the Spray-Dried Fexofenadine Zwitterion Dihydrate Formulation

The fexofenadine zwitterion dihydrate composition is then spray-dried to provide a spray-dried fexofenadine zwitterion dihydrate formulation, e.g., granules. One feature of the inventive processes is this formation of granules that contain fexofenadine zwitterion dihydrate. The granules produced according to these methods are not bitter tasting and, regardless of subsequent steps that are performed to the granules, the fexofenadine zwitterion dihydrate does not convert to fexofenadine hydrochloride. The granules comprise a spray-dried formulation containing fexofenadine zwitterion dihydrate that is obtained by spray-drying (i) a fexofenadine zwitterion dihydrate composition; and (ii) an inert matrix.

Spray-drying for use herein may be performed using a number of techniques and instruments. In some embodiments, the spray-drying is performed using a fluid bed and a spray system. The inventors found that a fluid bed combines the granulation and drying steps. The spray system provides granules that are well granulated. In some embodiments, the spray system is top spraying. In other embodiments, the spray system is bottom spraying. In further embodiments, the spray system is tangential.

The spray-drying is performed using an inert matrix. The components of the inert matrix include one or more pharmaceutically acceptable excipients. Desirably, the one or more pharmaceutically acceptable excipients maintain the fexofenadine zwitterion dihydrate form in the granules, *i.e.,* the fexofenadine zwitterion dihydrate does not convert to fexofenadine hydrochloride. In some embodiments, the pharmaceutically acceptable excipients maintain a pH of about 5.5 to about 7 so as to avoid the formation of fexofenadine hydrochloride. In other embodiments, the pharmaceutically acceptable excipients maintain a pH of about 5.5, about 5.6, about 5.7, about 5.8, about 5.9, about 6, about 6.1, about 6.2, about 6.3, about 6.4, about 6.5, about 6.6, about 6.7, about 6.8, about 6.9 or about 7. In other embodiments, the pharmaceutically acceptable excipient is a diluent, binder, glidant, or disintegrant, or a combination thereof.

Examples of diluents include, without limitation, microcrystalline cellulose, mannitol, maltitol, sucrose, erythritol, calcium phosphate dibasic, calcium phosphate tribasic, isomalt, xylitol, maltodextrin, lactose, starch, sorbitol, sucrose, a sucrose derivative, invert sucrose, lactitol, erythritol, fructose, glucose, calcium carbonate, cellulose, a cellulose derivative, calcium carbonate, calcium lactate, calcium sulfate, or calcium phosphate, or a combination thereof. In some aspects, the diluent is microcrystalline cellulose. In further aspects, the diluent is microcrystalline cellulose having a pH of about 5 to about 7.5, such as 6.2. In other aspects, the diluent is mannitol. Mannitol is available in the art as a variety of products. In yet other aspects, the diluent is mannitol having a pH if about 6 to about 7, such as 6.3. In further aspects, the diluent is maltitol. In further aspects, the diluent is maltitol having a pH of about 5 to about 7. In yet other aspects, the diluent is sucrose. In further aspects, the diluent is sucrose having a pH of about 7. In still further aspects, the diluent is erythritol. In further aspects, the diluent is erythritol having a pH of about 5 to about 7. In other aspects, the diluent is calcium phosphate dibasic. In further aspects, the diluent is calcium phosphate dibasic having a pH of about 5.1 to about 7.3, or such as about 7.3, or about 5.1, or about 6.1 to about 7.2. In further aspects, the diluent is calcium phosphate tribasic. In further aspects, the diluent is calcium phosphate tribasic having a pH of about 6.8. In still other aspects, the diluent is isomalt. In yet further aspects, the diluent is xylitol. In other aspects, the diluent is maltodextrin. In further aspects, the diluent is maltodextrin having a pH of about 4 to about 7. In further aspects, the diluent is lactose. In yet other aspects, the diluent is starch. In further embodiments, the diluent is starch having a pH of about 4 to about 8. In still further aspects, the diluent is sorbitol. In further aspects, the diluent is sorbitol having a pH of about 4.5 to about 7. In further aspects, the diluent is a sucrose derivative. In still other aspects, the diluent is invert sucrose. In yet further aspects, the diluent is lactitol. In further aspects, the diluent is lactitol having a pH of about 4.5 to about 7. In other aspects, the diluent is erythritol. In further aspects, the diluent is erythritol having a pH of about 5 to about 7. In further aspects, the diluent is fructose. In yet other aspects, the diluent is glucose. In further aspects, the diluent is glucose having a pH of about 4 to about 6. In still further aspects, the diluent is calcium carbonate. In further aspects, the diluent is calcium carbonate having a pH of about 9. In other aspects, the diluent is cellulose. In further aspects, the diluent is cellulose having a pH of about 5 to about 7.5 In further aspects, the diluent is a cellulose derivative. In yet other aspects, the diluent is calcium carbonate. In still further aspects, the diluent is calcium lactate. In other aspects, the diluent is calcium sulfate. In further aspects, the diluent is calcium phosphate. More than one diluent may be used to form the granules. In some embodiments, the granules are formed using one diluent. In other embodiments, the granules are formed using two diluents. In further embodiments, the granules are formed using three diluents.

Examples of disintegrants in the granules include, without limitation, sodium croscarmellose, sodium starch glycolate, crospovidone, starch pregelatinized, starch, or hydroxypropylcellulose (HPC), or a combination thereof. In some aspects, the disintegrant is sodium croscarmellose. In other aspects, the disintegrant is sodium starch glycolate. In further aspects, the disintegrant is crospovidone. In yet other aspects, the disintegrant is starch pregelatinized. In still further aspects, the disintegrant is starch. In other aspects, the disintegrant is hydroxypropylcellulose (HPC). More than one disintegrant may be used to form the granules. In some embodiments, the granules are formed using one disintegrant. In other embodiments, the granules are formed using two disintegrants. In further embodiments, the granules are formed using three disintegrants.

Examples of glidants in the granules include, without limitation, talc and silicon dioxide. In some aspects, the glidant is talc. In other aspects, the glidant is silicon dioxide. More than one glidant may be used to form the granules. In some embodiments, the granules are formed using one glidant. In other embodiments, the granules are formed using two glidants.

Examples of binders in the granules include, without limitation, hydroxypropylmethylcellulose (HPMC), hydroxypropylcellulose (HPC), polyvinylpyrrolidone (PVP), or carboxymethyl cellulose (CMC), hydroxyethylmethyl cellulose (HEMC), or hydroxyethylcellulose (HEC), or a combination thereof. In some aspects, the binder is HPMC. In other aspects, the binder is HPMC having a viscosity of about 4 to about 100,000 mPa.s. In further aspects, the binder is HPMC 4mPa.s or HPMC 5 mPa.s. In yet other aspects, the binder is HPC. In still further aspects, the binder is PVP. In other aspects, the binder is CMC. In further aspects, the binder is HEMC. In yet other aspects, the binder is HEC. More than one binder may be used to form the granules. In some embodiments, the granules are formed using one binder. In other embodiments, the granules are formed using two binders. In further embodiments, the granules are formed using three binders.

### C. Preparation of the Fexofenadine Zwitterion Dihydrate Final Drug Product

After formation of the spray-dried granules/formulation that contains the fexofenadine zwitterion dihydrate, additional steps may be performed to incorporate an external phase. In some embodiments, the external phase is included and integrates with the granules.

**In** some embodiments, an external phase is provided by combining the spray-dried formulation with one or more of a second portion of the disintegrant, a second disintegrant, a second portion of the diluent, a flavoring agent, a second portion of the wetting agent, one or more lubricants, or combination thereof.

In certain embodiments, the spray-dried formulation is combined with a second portion of the disintegrant that was used to prepare the granules. Examples of the disintegrant in the external phase include, without limitation, sodium croscarmellose, sodium starch glycolate, crospovidone, starch pregelatinized, starch, or hydroxypropylcellulose (HPC), or a combination thereof. In some aspects, the disintegrant in the external phase is sodium croscarmellose. In other aspects, the disintegrant in the external phase is sodium starch glycolate. In further aspects, the disintegrant in the external phase is crospovidone. In yet other aspects, the disintegrant in the external phase is starch pregelatinized. In still further aspects, the disintegrant in the external phase is starch. In other aspects, the disintegrant in the external phase is hydroxypropylcellulose (HPC).

In other embodiments, the spray-dried formulation is combined with a second disintegrant. Examples of the second disintegrant is sodium starch glycolate, crospovidone, starch pregelatinized, starch, hydroxypropylcellulose (HPC), or a combination thereof. In some aspects, the second disintegrant is sodium starch glycolate. In other aspects, the second disintegrant is crospovidone. In further aspects, the second disintegrant is starch pregelatinized. In yet other aspects, the second disintegrant is starch. In still further aspects, the second disintegrant is HPC.

In further aspects, the spray-dried formulation is combined with a second portion of the diluent that was used to prepare the granules. Examples of the diluent include, without limitation, microcrystalline cellulose, mannitol, maltitol, sucrose, erythritol, calcium phosphate dibasic, calcium phosphate tribasic, isomalt, xylitol, maltodextrin, lactose, starch, sorbitol, sucrose, a sucrose derivative, invert sucrose, lactitol, erythritol, fructose, glucose, calcium carbonate, cellulose, a cellulose derivative, calcium carbonate, calcium lactate, calcium sulfate, or calcium phosphate, or a combination thereof. In some aspects, the diluent is microcrystalline cellulose. In further aspects, the diluent is microcrystalline cellulose having a pH of about 5 to about 7.5, such as 6.2. In other aspects, the diluent is mannitol. In yet other aspects, the diluent is mannitol having a pH if about 6 to about 7, such as 6.3. In further aspects, the diluent is maltitol. In further aspects, the diluent is maltitol having a pH of about 5 to about 7. In yet other aspects, the diluent is sucrose. In further aspects, the diluent is sucrose having a pH of about 7. In still further aspects, the diluent is erythritol. In further aspects, the diluent is erythritol having a pH of about 5 to about 7. In other aspects, the diluent is calcium phosphate dibasic. In further aspects, the diluent is calcium phosphate dibasic having a pH of about 5.1 to about 7.3, or such as about 7.3, or about 5.1, or about 6.1 to about 7.2. In further aspects, the diluent is calcium phosphate tribasic. In further aspects, the diluent is calcium phosphate tribasic having a pH of about 6.8. In still other aspects, the diluent is isomalt. In yet further aspects, the diluent is xylitol. In other aspects, the diluent is maltodextrin. In further aspects, the diluent is maltodextrin having a pH of about 4 to about 7. In further aspects, the diluent is lactose. In yet other aspects, the diluent is starch. In further embodiments, the diluent is starch having a pH of about 4 to about 8. In still further aspects, the diluent is sorbitol. In further aspects, the diluent is sorbitol having a pH of about 4.5 to about 7. In further aspects, the diluent is a sucrose derivative. In still other aspects, the diluent is invert sucrose. In yet further aspects, the diluent is lactitol. In further aspects, the diluent is lactitol having a pH of about 4.5 to about 7. In other aspects, the diluent is erythritol. In further aspects, the diluent is erythritol having a pH of about 5 to about 7. In further aspects, the diluent is fructose. In yet other aspects, the diluent is glucose. In further aspects, the diluent is glucose having a pH of about 4 to about 6. In still further aspects, the diluent is calcium carbonate. In further aspects, the diluent is calcium carbonate having a pH of about 9. In other aspects, the diluent is cellulose. In further aspects, the diluent is cellulose having a pH of about 5to about 7.5 In further aspects, the diluent is a cellulose derivative. In yet other aspects, the diluent is calcium carbonate. In still further aspects, the diluent is calcium lactate. In other aspects, the diluent is calcium sulfate. In further aspects, the diluent is calcium phosphate.

In yet other aspects, the spray-dried formulation is combined with a flavoring agent that has been approved for use in humans by the U.S. FDA.

In still further aspects, the spray-dried formulation is combined with a second portion of the wetting agent. Examples of wetting agents include, without limitation, a poloxamer, polysorbate, or polyoxyl hydrogenated castor oil. In some embodiments, the wetting agent is a poloxamer such as poloxamer 188, poloxamer 237, poloxamer 338, poloxamer 407, or a combination thereof. In other embodiments, the wetting agent is a polysorbate such as polysorbate 20 (polyoxyethylene (20) sorbitan monolaurate), polysorbate 40 (polyoxyethylene (20) sorbitan monopalmitate), polysorbate 60 (polyoxyethylene (20) sorbitan monostearate), polysorbate 80 (polyoxyethylene (20) sorbitan monooleate), or SEPITRAP^{™} 80 (polysorbate 80 and magnesium aluminometasilicate). In further embodiments, the wetting agent is polysorbate 80 or SEPITRAP^{™} 80, or such as polysorbate 80, or such as SEPITRAP^{™} 80. In yet other embodiments, the wetting agent is a polyoxyl hydrogenated castor oil such as SEPITRAP^{™} 4000 (polyoxyl 40 hydrogenated castor oil and magnesium aluminosilicate).

In other aspects, the spray-dried formulation is combined with one or more lubricants. Examples of lubricants include, without limitation, polyethylene glycol (PEG), magnesium stearate, sodium stearyl fumarate, stearic acid, sorbitan monostearate, sucrose monopalmitate, glyceryl monostearate, glyceryl tribehenate, glyceryl dibehenate, calcium stearate, zinc stearate, hydrated magnesium silicate, sodium oleate, sterotex, sodium benzoate, talc, sodium acetate, a wax, sodium lauryl sulfate, or magnesium lauryl sulfate, or a combination thereof. In some aspects, the lubricant is PEG, such as a PEG greater than about 6000 (e.g., PEG 6000 PEG 8000, PEG 20000, or PEG 35000). In other aspects, the lubricant is magnesium stearate. In further aspects, the lubricant is sodium stearyl fumarate. In yet other aspects, the lubricant is stearic acid. In still further aspects, the lubricant is sorbitan monostearate. In other aspects, the lubricant is sucrose monopalmitate. In further aspects, the lubricant is glyceryl monostearate. In still other aspects, the lubricant is glyceryl tribehenate. In yet further aspects, the lubricant is glyceryl dibehenate. In other aspects, the lubricant is calcium stearate. In further aspects, the lubricant is zinc stearate. In yet other aspects, the lubricant is hydrated magnesium silicate. In still further aspects, the lubricant is sodium oleate. In other aspects, the lubricant is STEROTEX. In further aspects, the lubricant is sodium benzoate. In still other aspects, the lubricant is talc. In yet further aspects, the lubricant is sodium acetate. In other aspects, the lubricant is a wax. In further aspects, the lubricant is sodium lauryl sulfate. In yet other aspects, the lubricant is magnesium lauryl sulfate. In still further aspects, the lubricant comprises a PEG and a stearate, for example magnesium stearate. More than one lubricant may be used in the external phase. In some embodiments, the external phase uses one lubricants. In other embodiments, the external phase uses two lubricants. In further embodiments, the external phase uses three lubricants.

### Formulations / Compositions

The processes described herein provide products that are useful as described in the disclosure. In certain embodiments, the disclosure provides spray-dried formulations comprising fexofenadine zwitterion dihydrate and an inert matrix, *i.e.,* granules. The spray-dried formulation containing fexofenadine zwitterion dihydrate does not produce a bitter taste upon contact with a human subject's tongue. The term "bitter" as used herein refers to an aversive sharp, biting, and/or unpleasant taste that occurs when a substance comes into contact with tastebuds or tongue of a subject.

Further, the fexofenadine zwitterion products defined herein (formulations and compositions) may be chewed or swallowed. In some aspects, chewing provide a faster availability for the fexofenadine to be absorbed, as compared to a swallowed tablet which must fully disintegrate before the fexofenadine can be absorbed. Thus, fexofenadine zwitterion may be formulated in both a chewable form and a swallowable form.

As discussed above, these spray-dried formulations are in the form of granules and provide a stable medium for the fexofenadine zwitterion dihydrate. That is, the fexofenadine zwitterion dihydrate stays in the form of the zwitterion in the granules and does not convert to fexofenadine hydrochloride or other fexofenadine salt with combined with other excipients. In some embodiments, the fexofenadine zwitterion dihydrate stays in the form of the zwitterion in the granules when formulated as a solid dosage form.

The term "inert matrix" as used herein refers to a solid form that provides a stable environment for the fexofenadine zwitterion dihydrate. In some embodiments, the inert matrix comprises one or more pharmaceutically acceptable excipients. The one or more pharmaceutically acceptable excipients is a diluent or a first disintegrant.

Examples of diluents include, without limitation, microcrystalline cellulose, mannitol, maltitol, sucrose, erythritol, calcium phosphate dibasic, calcium phosphate tribasic, isomalt, xylitol, maltodextrin, lactose, starch, sorbitol, sucrose, a sucrose derivative, invert sucrose, lactitol, erythritol, fructose, glucose, calcium carbonate, cellulose, a cellulose derivative, calcium carbonate, calcium lactate, calcium sulfate, or calcium phosphate, or a combination thereof. In some aspects, the diluent is microcrystalline cellulose. In further aspects, the diluent is microcrystalline cellulose having a pH of about 5 to about 7.5, such as 6.2. In other aspects, the diluent is mannitol. In yet other aspects, the diluent is mannitol having a pH if about 6 to about 7, such as 6.3. In further aspects, the diluent is maltitol. In further aspects, the diluent is maltitol having a pH of about 5 to about 7. In yet other aspects, the diluent is sucrose. In further aspects, the diluent is sucrose having a pH of about 7. In still further aspects, the diluent is erythritol. In further aspects, the diluent is erythritol having a pH of about 5 to about 7. In other aspects, the diluent is calcium phosphate dibasic. In further aspects, the diluent is calcium phosphate dibasic having a pH of about 5.1 to about 7.3, or such as about 7.3, or about 5.1, or about 6.1 to about 7.2. In further aspects, the diluent is calcium phosphate tribasic. In further aspects, the diluent is calcium phosphate tribasic having a pH of about 6.8. In still other aspects, the diluent is isomalt. In yet further aspects, the diluent is xylitol. In other aspects, the diluent is maltodextrin. In further aspects, the diluent is maltodextrin having a pH of about 4 to about 7. In further aspects, the diluent is lactose. In yet other aspects, the diluent is starch. In further embodiments, the diluent is starch having a pH of about 4 to about 8. In still further aspects, the diluent is sorbitol. In further aspects, the diluent is sorbitol having a pH of about 4.5 to about 7. In further aspects, the diluent is a sucrose derivative. In still other aspects, the diluent is invert sucrose. In yet further aspects, the diluent is lactitol. In further aspects, the diluent is lactitol having a pH of about 4.5 to about 7. In other aspects, the diluent is erythritol. In further aspects, the diluent is fructose. In yet other aspects, the diluent is glucose. In further aspects, the diluent is glucose having a pH of about 4 to about 6. In still further aspects, the diluent is calcium carbonate. In further aspects, the diluent is calcium carbonate having a pH of about 9. In other aspects, the diluent is cellulose. In further aspects, the diluent is cellulose having a pH of about 5to about 7.5 In further aspects, the diluent is a cellulose derivative. In yet other aspects, the diluent is calcium carbonate. In still further aspects, the diluent is calcium lactate. In other aspects, the diluent is calcium sulfate. In further aspects, the diluent is calcium phosphate.

Examples of the first disintegrant include sodium croscarmellose, sodium starch glycolate, crospovidone, starch pregelatinized, starch, hydroxypropylcellulose (HPC), or a combination thereof. In some aspects, the first disintegrant is sodium croscarmellose. In other aspects, the first disintegrant is sodium starch glycolate. In further aspects, the first disintegrant is crospovidone. In yet other aspects, the first disintegrant is starch pregelatinized. In still further aspects, the first disintegrant is starch. In other aspects, the first disintegrant is hydroxypropylcellulose (HPC).

The spray-dried formulation/granules may be combined with additional excipients to provide a final fexofenadine zwitterion dihydrate composition. In some embodiments, the spray-dried formulation further contains one or more of a wetting agent, alkalizing agent, buffer, sweetener, binder, or solvent.

In other embodiments, the spray-dried formulation/granules further contains one or more of a wetting agent. Examples of wetting agents include, without limitation, a poloxamer, polysorbate, or polyoxyl hydrogenated castor oil. In some embodiments, the wetting agent is a poloxamer such as poloxamer 188, poloxamer 237, poloxamer 338, poloxamer 407, or a combination thereof. In other embodiments, the wetting agent is a polysorbate such as polysorbate 20 (polyoxyethylene (20) sorbitan monolaurate), polysorbate 40 (polyoxyethylene (20) sorbitan monopalmitate), polysorbate 60 (polyoxyethylene (20) sorbitan monostearate), polysorbate 80 (polyoxyethylene (20) sorbitan monooleate), or SEPITRAP^{™} 80 (polysorbate 80 and magnesium aluminometasilicate). In further embodiments, the wetting agent is polysorbate 80 or SEPITRAP^{™} 80, or such as polysorbate 80, or such as SEPITRAP^{™} 80. In yet other embodiments, the wetting agent is a polyoxyl hydrogenated castor oil such as SEPITRAP^{™} 4000 (polyoxyl 40 hydrogenated castor oil and magnesium aluminosilicate).

In further embodiments, the spray-dried formulation/granules further contains one or more of an alkalizing agent. In certain aspects, the alkalizing agent is a strong base. In some aspects, the alkalizing agent is potassium hydroxide, calcium hydroxide, sodium hydroxide, or barium hydroxide, among others. In other aspects, alkalizing agent is potassium hydroxide. In further aspects, the alkalizing agent is calcium hydroxide. In yet other aspects, the alkalizing agent is sodium hydroxide. In still further aspects, the alkalizing agent is barium hydroxide.

In yet other embodiments, the spray-dried formulation/granules further contains one or more of a buffer. Examples of buffers include, without limitation, sodium phosphate dibasic 2H₂O, sodium phosphate monobasic, citric acid/sodium phosphate dibasic, sodium phosphate dibasic hydrate, succinic acid/sodium hydroxide, citric acid/sodium citrate, sodium citrate hydrate, potassium citrate, maleic acid/sodium hydroxide, fumaric acid/sodium hydroxide, sodium phosphate monobasic hydrate, potassium phosphate monobasic/sodium phosphate dibasic, sodium phosphate dibasic hydrate, potassium phosphate dibasic, potassium phosphate dibasic hydrate. In some embodiments, the buffer is sodium phosphate monobasic, sodium phosphate monobasic hydrate, potassium phosphate monobasic/sodium phosphate dibasic, sodium phosphate dibasic hydrate, potassium phosphate dibasic, or potassium phosphate dibasic hydrate. In other embodiments, the buffer is sodium phosphate monobasic, sodium phosphate monobasic hydrate/sodium phosphate dibasic, or sodium phosphate dibasic hydrate, or a combination thereof. In further embodiments, the buffer is phosphate dibasic and phosphate monobasic.

In still further embodiments, the spray-dried formulation/granules further contains one or more of a sweetener. Examples of sweeteners include sucralose, aspartame, acesulfame potassium, saccharin, steviol glycosides, neotame, advantame, saccharin sodium, cyclamate sodium, or ammonium glycyrrhizate, or a combination thereof. In some aspects, the sweetener is sucralose. In other aspects, the sweetener is aspartame. In further aspects, the sweetener is acesulfame potassium. In yet other aspects, the sweetener is saccharin. In still further aspects, the sweetener is a steviol glycoside. In other aspects, the sweetener is neotame. In further aspects, the sweetener is advantame. In yet other aspects, the sweetener is saccharin sodium. In still further aspects, the sweetener is cyclamate sodium. In other aspects, the sweetener is ammonium glycyrrhizate.

In other embodiments, the spray-dried formulation/granules further contains one or more of a binder. Examples of binders include, without limitation, hydroxypropylmethylcellulose (HPMC), hydroxypropylcellulose (HPC), polyvinylpyrrolidone (PVP), or carboxymethyl cellulose (CMC), hydroxyethylmethyl cellulose (HEMC), or hydroxyethylcellulose (HEC), or a combination thereof. In some aspects, the binder is HPMC. In other aspects, the binder is HPMC having a viscosity of about 4 to about 100,000 mPa.s. In further aspects, the binder is HPMC 4mPa.s or HPMC 5 mPa.s. In yet other aspects, the binder is HPC. In still further aspects, the binder is PVP. In other aspects, the binder is CMC. In further aspects, the binder is HEMC. In yet other aspects, the binder is HEC.

In further embodiments, the spray-dried formulation/granules further contains one or more of a solvent. In some embodiments, the solvent is water. In other embodiments, the solvent is sterile water.

The spray-dried formulation/granules may be combined with additional excipients to provide a finished products comprising a disclosed fexofenadine zwitterion composition. In some embodiments, the final fexofenadine zwitterion composition further contains one or more of a second disintegrant, flavoring agent, and lubricant. In some embodiments, the spray-dried formulation contains a second disintegrant. Examples of the second disintegrant include sodium starch glycolate, crospovidone, starch pregelatinized, starch, or hydroxypropylcellulose (HPC), or a combination thereof. In some aspects, the second disintegrant is sodium starch glycolate. In further aspects, the second disintegrant is crospovidone. In other aspects, the second disintegrant is starch pregelatinized. In still further aspects, the second disintegrant is starch. In other aspects, the second disintegrant is hydroxypropylcellulose (HPC).

In other embodiments, the final fexofenadine composition contains a flavoring agent.

In further embodiments, the final fexofenadine composition contains a lubricant. Examples of lubricants include, without limitation, polyethylene glycol (PEG), magnesium stearate, sodium stearyl fumarate, stearic acid, sorbitan monostearate, sucrose monopalmitate, glyceryl monostearate, glyceryl tribehenate, glyceryl dibehenate, calcium stearate, and zinc stearate, hydrated magnesium silicate, sodium oleate, sterotex, sodium benzoate, talc, sodium acetate, a wax, sodium lauryl sulfate, or magnesium lauryl sulfate, or a combination thereof. In some aspects, the lubricant is PEG, such as PEG 6000. In other aspects, the lubricant is magnesium stearate. In further aspects, the lubricant is sodium stearyl fumarate. In yet other aspects, the lubricant is stearic acid. In still further aspects, the lubricant is sorbitan monostearate. In other aspects, the lubricant is sucrose monopalmitate. In further aspects, the lubricant is glyceryl monostearate. In still other aspects, the lubricant is glyceryl tribehenate. In yet further aspects, the lubricant is glyceryl dibehenate. In other aspects, the lubricant is calcium stearate. In further aspects, the lubricant is zinc stearate. In yet other aspects, the lubricant is hydrated magnesium silicate. In still further aspects, the lubricant is sodium oleate. In other aspects, the lubricant is STEROTEX. In further aspects, the lubricant is sodium benzoate. In still other aspects, the lubricant is talc. In yet further aspects, the lubricant is sodium acetate. In other aspects, the lubricant is a wax. In further aspects, the lubricant is sodium lauryl sulfate. In yet other aspects, the lubricant is magnesium lauryl sulfate. In still further aspects, the lubricant comprises a PEG and a stearate, for example magnesium stearate.

In certain aspects, the disclosure provides a spray-dried formulation, comprising fexofenadine zwitterion dihydrate, a wetting agent, an alkalizing agent, a buffer, a sweetener, a binder, a diluent, a disintegrant, a flavoring agent, and a lubricant. In some embodiments, the spray-dried formulation contains two wetting agents. Examples of wettings without limitation, a poloxamer, polysorbate, or polyoxyl hydrogenated castor oil. In some embodiments, the wetting agent is a poloxamer such as poloxamer 188, poloxamer 237, poloxamer 338, poloxamer 407, or a combination thereof. In other embodiments, the wetting agent is a polysorbate such as polysorbate 20 (polyoxyethylene (20) sorbitan monolaurate), polysorbate 40 (polyoxyethylene (20) sorbitan monopalmitate), polysorbate 60 (polyoxyethylene (20) sorbitan monostearate), polysorbate 80 (polyoxyethylene (20) sorbitan monooleate), or SEPITRAP^{™} 80 (polysorbate 80 and magnesium aluminometasilicate). In further embodiments, the wetting agent is polysorbate 80 or SEPITRAP^{™} 80, or such as polysorbate 80, or such as SEPITRAP^{™} 80. In yet other embodiments, the wetting agent is a polyoxyl hydrogenated castor oil such as SEPITRAP^{™} 4000 (polyoxyl 40 hydrogenated castor oil and magnesium aluminosilicate). The spray-dried formulation may contain about 0.5 to about 10% w/w of the one or more wetting agent. In some embodiments, the spray dried formulation contains about 0.5, about 1, about 2, about 3, about 4, about 5, about 6, about 7, about 8, about 9, or about 10% w/w of the one or more wetting agent. In other embodiments, the spray-dried formulation contains about 0.5 to about 9, about 0.5 to about 8, about 0.5 to about 7, about 0.5 to about 6, about 0.5 to about 5, about 0.5 to about 4, about 0.5 to about 3, about 0.5 to about 2, about 0.5 to about 1, about 1 to about 10, about 1 to about 9, about 1 to about 8, about 1 to about 7, about 1 to about 6, about 1 to about 5, about 1 to about 4, about 1 to about 3, about 1 to about 2, about 2 to about 10, about 2 to about 9, about 2 to about 8, about 2 to about 7, about 2 to about 6, about 2 to about 5, about 2 to about 4, about 2 to about 3, about 3 to about 10, about 3 to about 9, about 3 to about 8, about 3 to about 7, about 3 to about 6, about 3 to about 5, about 3 to about 4, about 4 to about 10, about 4 to about 9, about 4 to about 8, about 4 to about 7, about 4 to about 6, about 4 to about 5, about 5 to about 10, about 5 to about 9, about 5 to about 8, about 5 to about 7, about 5 to about 6, about 6 to about 10, about 6 to about 9, about 6 to about 8, about 6 to about 7, about 7 to about 10, about 7 to about 9, about 7 to about 8, about 8 to about 10, about 8 to about 9, or about 9 to about 10% w/w of the one or more wetting agent.

In other embodiments, the spray-dried formulation contains an alkalizing agent. In certain aspects, the alkalizing agent is a strong base. In some aspects, the alkalizing agent is potassium hydroxide, calcium hydroxide, sodium hydroxide, or barium hydroxide, among others. In other aspects, alkalizing agent is potassium hydroxide. In further aspects, the alkalizing agent is calcium hydroxide. In yet other aspects, the alkalizing agent is sodium hydroxide. In still further aspects, the alkalizing agent is barium hydroxide. The spray-dried formulation may contain about 0.5 to about 5% w/w of the one or more alkalizing agent. In some embodiments, the spray-dried formulation contains about 0.5, about 1, about 1.5, about 2, about 2.5, about 3, about 3.5, about 4, about 4.5, or about 5% w/w of the one or more alkalizing agent. In other embodiments, the spray-dried formulation contains about 0.5 to about 5, about 0.5 to about 4.5, about 0.5 to about 4, about 0.5 to about 3.5, about 0.5 to about 3, about 0.5 to about 2.5, about 0.5 to about 2, about 0.5 to about 1.5, about 0.5 to about 1, about 1 to about 5, about 1 to about 4.5, about 1 to about 4, about 1 to about 3.5, about 1 to about 3, about 1 to about 2.5, about 1 to about 2, about 1 to about 1.5, about 1.5 to about 5, about 1.5 to about 4.5, about 1.5 to about 4, about 1.5 to about 3.5, about 1.5 to about 3, about 1.5 to about 2.5, about 1.5 to about 2, about 2 to about 5, about 2 to about 4.5, about 2 to about 4, about 2 to about 3.5, about 2 to about 3, about 2 to about 2.5, about 2.5 to about 5, about 2.5 to about 4.5, about 2.5 to about 4, about 2.5 to about 3.5, about 2.5 to about 3, about 3 to about 5, about 3 to about 4.5, about 3 to about 4, about 3 to about 3.5, about 3.5 to about 5, about 3.5 to about 4.5, about 3.5 to about 4, about 4 to about 5, about 4 to about 4.5, or about 4.5 to about 5% w/w of the one or more alkalizing agent.

In further embodiments, the spray-dried formulation contains one or two buffers. In some aspects, the spray-dried formulation contains one buffer. In other aspects, the spray-dried formulation contains two buffers. Examples of buffers include, without limitation, sodium phosphate dibasic 2H₂O, sodium phosphate monobasic, citric acid/sodium phosphate dibasic, sodium phosphate dibasic hydrate, succinic acid/sodium hydroxide, citric acid/sodium citrate, sodium citrate hydrate, potassium citrate, maleic acid/sodium hydroxide, fumaric acid/sodium hydroxide, sodium phosphate monobasic hydrate, potassium phosphate monobasic/sodium phosphate dibasic, sodium phosphate dibasic hydrate, potassium phosphate dibasic, potassium phosphate dibasic hydrate. In some embodiments, the buffer is sodium phosphate monobasic, sodium phosphate monobasic hydrate, potassium phosphate monobasic/sodium phosphate dibasic, sodium phosphate dibasic hydrate, potassium phosphate dibasic, or potassium phosphate dibasic hydrate. In other embodiments, the buffer is sodium phosphate monobasic, sodium phosphate monobasic hydrate/sodium phosphate dibasic, or sodium phosphate dibasic hydrate, or a combination thereof. In further embodiments, the buffer is phosphate dibasic and phosphate monobasic. In some aspects, the buffer is a sodium phosphate hydrate, such as sodium phosphate dibasic 2H₂O, sodium phosphate monobasic, or a combination thereof. The spray-dried formulation contains about 0.2 to about 10% w/w, of the one or more buffer. In some embodiments, the spray dried formulation contains about 0.2, about 0.5, about 1, about 2, about 3, about 4, about 5, about 6, about 7, about 8, about 9, or about 10% w/w of the one or more buffer. In other embodiments, the spray-dried formulation contains 0.2 to about 9, about 0.2 to about 8, about 0.2 to about 7, about 0.2 to about 6, about 0.2 to about 5, about 0.2 to about 4, about 0.2 to about 3, about 0.2 to about 2, about 0.2 to about 1, about 0.5 to about 10, about 0.5 to about 9, about 0.5 to about 8, about 0.5 to about 7, about 0.5 to about 6, about 0.5 to about 5, about 0.5 to about 4, about 0.5 to about 3, about 0.5 to about 2, about 0.5 to about 1, about 1 to about 10, about 1 to about 9, about 1 to about 8, about 1 to about 7, about 1 to about 6, about 1 to about 5, about 1 to about 4, about 1 to about 3, about 1 to about 2, about 2 to about 10, about 2 to about 9, about 2 to about 8, about 2 to about 7, about 2 to about 6, about 2 to about 5, about 2 to about 4, about 2 to about 3, about 3 to about 10, about 3 to about 9, about 3 to about 8, about 3 to about 7, about 3 to about 6, about 3 to about 5, about 3 to about 4, about 4 to about 10, about 4 to about 9, about 4 to about 8, about 4 to about 7, about 4 to about 6, about 4 to about 5, about 5 to about 10, about 5 to about 9, about 5 to about 8, about 5 to about 7, about 5 to about 6, about 6 to about 10, about 6 to about 9, about 6 to about 8, about 6 to about 7, about 7 to about 10, about 7 to about 9, about 7 to about 8, about 8 to about 10, about 8 to about 9, or about 9 to about 10% w/w of the one or more buffer.

Examples of sweeteners include sucralose, aspartame, acesulfame potassium, saccharin, steviol glycosides, neotame, advantame, saccharin sodium, cyclamate sodium, or ammonium glycyrrhizate, or a combination thereof. In some aspects, the sweetener is sucralose. In other aspects, the sweetener is aspartame. In further aspects, the sweetener is acesulfame potassium. In yet other aspects, the sweetener is saccharin. In still further aspects, the sweetener is a steviol glycoside. In other aspects, the sweetener is neotame. In further aspects, the sweetener is advantame. In yet other aspects, the sweetener is saccharin sodium. In still further aspects, the sweetener is cyclamate sodium. The spray-dried formulation contains about 0.2 to about 3% w/w of the one or more sweetener. In some aspects, the spray-dried formulation contains about 0.2, about 0.5, about 0.75, about 1, about 1.25, about 1.5, about 1.75, about 2, about 2.25, about 2.5, about 2.75, about or 3% w/w of the one or more sweetener. In other aspects, the spray-dried formulation contains 0.2 to about 2.5, about 0.2 to about 2, about 0.2 to about 1.5, about 0.2 to about 1, about 0.2 to about 0.5, about 0.5 to about 3, about 0.5 to about 2.5, about 0.5 to about 2, about 0.5 to about 1.5, about 0.5 to about 1, about 1 to about 3, about 1 to about 2.5, about 1 to about 2, about 1 to about 1.5, about 1.5 to about 3, about 1.5 to about 2.5, about 1.5 to about 2, about 2 to about 3, about 2 to about 2.5, or about 2.5 to about 3% w/w of the one or more sweetener.

In yet other embodiments, the spray-dried formulation contains a binder. Examples of binders include, without limitation, hydroxypropylmethylcellulose (HPMC), hydroxypropylcellulose (HPC), polyvinylpyrrolidone (PVP), or carboxymethyl cellulose (CMC), hydroxyethylmethyl cellulose (HEMC), or hydroxyethylcellulose (HEC), or a combination thereof. In some aspects, the binder is HPMC. In other aspects, the binder is HPMC having a viscosity of about 4 to about 100,000 mPa.s. In further aspects, the binder is HPMC 4mPa.s or HPMC 5 mPa.s. In yet other aspects, the binder is HPC. In still further aspects, the binder is PVP. In other aspects, the binder is CMC. In further aspects, the binder is HEMC. In yet other aspects, the binder is HEC. The spray-dried formulation may contain about 0.5 to about 5% w/w of the one or more binder. In some embodiments, the spray dried formulation contains about 0.5, about 1, about 2, about 3, about 4, or about 5% w/w of the one or more binder. In other embodiments, the spray-dried formulation contains about 0.5 to about 5, about 0.5 to about 4, about 0.5 to about 3, about 0.5 to about 2, about 0.5 to about 1, about 1 to about 5, about 1 to about 4, about 1 to about 3, about 1 to about 2, about 2 to about 5, about 2 to about 4, about 2 to about 3, about 3 to about 5, about 3 to about 4, or about 4 to about 5% w/w of the one or more binder.

In still further embodiments, the spray-dried formulation contains one or more diluent. In some aspects, the spray-dried formulation contains one, two, three, four, five, or six diluents. In other aspects, the spray-dried formulation contains two diluents. In further aspects, the spray-dried formulation contains three diluents. In yet other aspects, the spray-dried formulation contains four diluents. In still further aspects, the spray-dried formulation contains five diluents. In other aspects, the spray-dried formulation contains six diluents. Examples of diluents include, without limitation, microcrystalline cellulose, mannitol, maltitol, sucrose, erythritol, calcium phosphate dibasic, calcium phosphate tribasic, isomalt, xylitol, maltodextrin, lactose, starch, sorbitol, sucrose, a sucrose derivative, invert sucrose, lactitol, erythritol, fructose, glucose, calcium carbonate, cellulose, a cellulose derivative, calcium carbonate, calcium lactate, calcium sulfate, or calcium phosphate, or a combination thereof. In certain aspects, the diluent is microcrystalline cellulose. In further aspects, the diluent is microcrystalline cellulose having a pH of about 5 to about 7.5, such as 6.2. In other aspects, the diluent is mannitol. In yet other aspects, the diluent is mannitol having a pH if about 6 to about 7, such as 6.3. In further aspects, the diluent is maltitol. In further aspects, the diluent is maltitol having a pH of about 5 to about 7. In yet other aspects, the diluent is sucrose. In further aspects, the diluent is sucrose having a pH of about 7. In still further aspects, the diluent is erythritol. In further aspects, the diluent is erythritol having a pH of about 5 to about 7. In other aspects, the diluent is calcium phosphate dibasic. In further aspects, the diluent is calcium phosphate dibasic having a pH of about 5.1 to about 7.3, or such as about 7.3, or about 5.1, or about 6.1 to about 7.2. In further aspects, the diluent is calcium phosphate tribasic. In further aspects, the diluent is calcium phosphate tribasic having a pH of about 6.8. In still other aspects, the diluent is isomalt. In yet further aspects, the diluent is xylitol. In other aspects, the diluent is maltodextrin. In further aspects, the diluent is maltodextrin having a pH of about 4 to about 7. In further aspects, the diluent is lactose. In yet other aspects, the diluent is starch. In further embodiments, the diluent is starch having a pH of about 4 to about 8. In still further aspects, the diluent is sorbitol. In further aspects, the diluent is sorbitol having a pH of about 4.5 to about 7. In further aspects, the diluent is a sucrose derivative. In still other aspects, the diluent is invert sucrose. In yet further aspects, the diluent is lactitol. In further aspects, the diluent is lactitol having a pH of about 4.5 to about 7. In other aspects, the diluent is erythritol. In further aspects, the diluent is erythritol having a pH of about 5 to about 7. In further aspects, the diluent is fructose. In yet other aspects, the diluent is glucose. In further aspects, the diluent is glucose having a pH of about 4 to about 6. In still further aspects, the diluent is calcium carbonate. In further aspects, the diluent is calcium carbonate having a pH of about 9. In other aspects, the diluent is cellulose. In further aspects, the diluent is cellulose having a pH of about 5 to about 7.5 In further aspects, the diluent is a cellulose derivative. In yet other aspects, the diluent is calcium carbonate. In still further aspects, the diluent is calcium lactate. In other aspects, the diluent is calcium sulfate. In further aspects, the diluent is calcium phosphate. The spray-dried formulation may contain about 30 to about 80% w/w of the one or more diluent. In some aspects, the spray-dried formulation contains about 30, about 40, about 50, about 60, about 70, or about 80% w/w of the one or more diluent. In other aspects, the spray-dried formulation contains about 30 to about 70, about 30 to about 60, about 30 to about 50, about 30 to about 40, about 40 to about 80, about 40 to about 70, about 40 to about 60, about 40 to about 50, about 50 to about 80, about 50 to about 70, about 50 to about 60, about 60 to about 80, about 60 to about 70, or about 70 to about 80% w/w of the one or more diluent.

In further embodiments, the spray-dried formulation contains one or more of a disintegrant. In some aspects the spray-dried formulation contains two disintegrants. In other aspects, the spray-dried formulation contains three disintegrants. Examples of disintegrants include, without limitation, sodium croscarmellose, sodium starch glycolate, crospovidone, starch pregelatinized, starch, or hydroxypropylcellulose (HPC), or a combination thereof. In some aspects, the disintegrant is sodium croscarmellose. In other aspects, the disintegrant is sodium starch glycolate. In further aspects, the disintegrant is crospovidone. In yet other aspects, the disintegrant is starch pregelatinized. In still further aspects, the disintegrant is starch. In other aspects, the disintegrant is hydroxypropylcellulose (HPC). The spray-dried formulation may contain about 3 to about 15% w/w of the one or more disintegrant. In certain aspects, the spray-dried formulation contains about 3, about 4, about 5, about 6, about 7, about 8, about 9, about 10, about 11, about 12, about 13, about 14, or about 15% w/w of the one or more disintegrants. In other aspects, the spray-dried formulation contains about 3 to about 14, about 3 to about 13, about 3 to about 12, about 3 to about 11, about 3 to about 10, about 3 to about 9, about 3 to about 8, about 3 to about 7, about 3 to about 6, about 3 to about 5, about 3 to about 4, about 4 to about 15, about 4 to about 14, about 4 to about 13, about 4 to about 12, about 4 to about 11, about 4 to about 10, about 4 to about 9, about 4 to about 8, about 4 to about 7, about 4 to about 6, about 4 to about 5, about 5 to about 15, about 5 to about 14, about 5 to about 13, about 5 to about 12, about 5 to about 11, about 5 to about 10, about 5 to about 9, about 5 to about 8, about 5 to about 7, about 5 to about 6, about 6 to about 15, about 6 to about 14, about 6 to about 13, about 6 to about 12, about 6 to about 11, about 6 to about 10, about 6 to about 9, about 6 to about 8, about 6 to about 7, about 7 to about 15, about 7 to about 14, about 7 to about 13, about 7 to about 12, about 7 to about 11, about 7 to about 10, about 7 to about 9, about 7 to about 8, about 8 to about 15, about 8 to about 14, about 8 to about 13, about 8 to about 12, about 8 to about 11, about 8 to about 10, about 8 to about 9, about 9 to about 15, about 9 to about 14, about 9 to about 13, about 9 to about 12, about 9 to about 11, about 9 to about 10, about 10 to about 15, about 10 to about 14, about 10 to about 13, about 10 to about 12, about 10 to about 11, about 11 to about 15, about 11 to about 14, about 11 to about 13, about 11 to about 12, about 12 to about 15, about 12 to about 14, about 12 to about 13, about 13 to about 15, about 13 to about 14, or about 14 to about 15% w/w of the one or more disintegrants.

In yet other embodiments, the spray-dried formulation contains one or more lubricants. In some aspects, the spray-dried formulation contains two lubricants. Examples of lubricants include, without limitation, polyethylene glycol (PEG), magnesium stearate, sodium stearyl fumarate, stearic acid, sorbitan monostearate, sucrose monopalmitate, glyceryl monostearate, glyceryl tribehenate, glyceryl dibehenate, calcium stearate, and zinc stearate, hydrated magnesium silicate, sodium oleate, sterotex, sodium benzoate, talc, sodium acetate, a wax, sodium lauryl sulfate, or magnesium lauryl sulfate, or a combination thereof. In some aspects, the lubricant is PEG, such as PEG 6000. In other aspects, the lubricant is magnesium stearate. In further aspects, the lubricant is sodium stearyl fumarate. In yet other aspects, the lubricant is stearic acid. In still further aspects, the lubricant is sorbitan monostearate. In other aspects, the lubricant is sucrose monopalmitate. In further aspects, the lubricant is glyceryl monostearate. In still other aspects, the lubricant is glyceryl tribehenate. In yet further aspects, the lubricant is glyceryl dibehenate. In other aspects, the lubricant is calcium stearate. In further aspects, the lubricant is zinc stearate. In yet other aspects, the lubricant is hydrated magnesium silicate. In still further aspects, the lubricant is sodium oleate. In other aspects, the lubricant is STEROTEX. In further aspects, the lubricant is sodium benzoate. In still other aspects, the lubricant is talc. In yet further aspects, the lubricant is sodium acetate. In other aspects, the lubricant is a wax. In further aspects, the lubricant is sodium lauryl sulfate. In yet other aspects, the lubricant is magnesium lauryl sulfate. In still further aspects, the lubricant comprises a PEG and a stearate, for example magnesium stearate. The spray-dried formulation may contain about 0.5 to about 10% w/w of the one or more lubricant. In some embodiments, the spray dried formulation contains about 0.5, about 1, about 2, about 3, about 4, about 5, about 6, about 7, about 8, about 9, or about 10% w/w of the one or more lubricant. In other embodiments, the spray-dried formulation contains about 0.5 to about 9, about 0.5 to about 8, about 0.5 to about 7, about 0.5 to about 6, about 0.5 to about 5, about 0.5 to about 4, about 0.5 to about 3, about 0.5 to about 2, about 0.5 to about 1, about 1 to about 10, about 1 to about 9, about 1 to about 8, about 1 to about 7, about 1 to about 6, about 1 to about 5, about 1 to about 4, about 1 to about 3, about 1 to about 2, about 2 to about 10, about 2 to about 9, about 2 to about 8, about 2 to about 7, about 2 to about 6, about 2 to about 5, about 2 to about 4, about 2 to about 3, about 3 to about 10, about 3 to about 9, about 3 to about 8, about 3 to about 7, about 3 to about 6, about 3 to about 5, about 3 to about 4, about 4 to about 10, about 4 to about 9, about 4 to about 8, about 4 to about 7, about 4 to about 6, about 4 to about 5, about 5 to about 10, about 5 to about 9, about 5 to about 8, about 5 to about 7, about 5 to about 6, about 6 to about 10, about 6 to about 9, about 6 to about 8, about 6 to about 7, about 7 to about 10, about 7 to about 9, about 7 to about 8, about 8 to about 10, about 8 to about 9, or about 9 to about 10% w/w of the one or more lubricant.

In still further embodiments, the spray-dried formulation may contain one or more of a flavoring agent. Examples of flavoring agents include agents having grape or mint flavors. The spray-dried formulation may contain about 0.1 to about 5% w/w of the one or more flavoring agent. In some aspects, the spray dried formulation contains about 0.5, about 1, about 2, about 3, about 4, or about 5% w/w of the one or more flavoring agent. In other aspects, the spray-dried formulation contains about 0.5 to about 5, about 0.5 to about 4, about 0.5 to about 3, about 0.5 to about 2, about 0.5 to about 1, about 1 to about 5, about 1 to about 4, about 1 to about 3, about 1 to about 2, about 2 to about 5, about 2 to about 4, about 2 to about 3, about 3 to about 5, about 3 to about 4, or about 4 to about 5% w/w of the one or more flavoring agent.

In other embodiments, the spray-dried formulation contains fexofenadine zwitterionic dihydrate, a wetting agent, an alkalizing agent, a first buffer, a second buffer, a sweetener, a binder, a first diluent, a second diluent, a first disintegrant, a second disintegrant, a flavoring agent, and a lubricant.

In further embodiments, the spray-dried formulation contains fexofenadine zwitterionic dihydrate, a wetting agent, an alkalizing agent, a first buffer, a second buffer, a sweetener, a binder, a first diluent, a second diluent, a first disintegrant, a second disintegrant, a first lubricant, a second lubricant, and a flavoring agent.

In yet other embodiments, the spray-dried formulation contains fexofenadine zwitterionic dihydrate, a wetting agent, an alkalizing agent, a first buffer, a second buffer, a sweetener, a binder, a first diluent, a second diluent, a third diluent, a first disintegrant, a second disintegrant, a flavoring agent, and a lubricant.

In still further embodiments, the spray-dried formulation contains fexofenadine zwitterionic dihydrate, a wetting agent, an alkalizing agent, a first buffer, a second buffer, a sweetener, a binder, a first diluent, a second diluent, a first disintegrant, a second disintegrant, a third disintegrant, a flavoring agent, and a lubricant.

In other embodiments, the spray-dried formulation contains fexofenadine zwitterionic dihydrate, a wetting agent, an alkalizing agent, a first buffer, a second buffer, a sweetener, a binder, a first diluent, a second diluent, a third diluent, a first disintegrant, a second disintegrant, a third disintegrant, a flavoring agent, and a lubricant.

In further embodiments, the spray-dried formulation contains fexofenadine zwitterionic dihydrate, a first wetting agent, a second wetting agent, an alkalizing agent, a first buffer, a second buffer, a sweetener, a binder, a first diluent, a second diluent, a third diluent, a first disintegrant, a second disintegrant, a third disintegrant, a flavoring agent, and a lubricant.

In still other embodiments, the spray-dried formulation contains fexofenadine zwitterionic dihydrate, a wetting agent, an alkalizing agent, a first buffer, a second buffer, a sweetener, a binder, a first diluent, a second diluent, a third diluent, a fourth diluent, a fifth diluent, a first disintegrant, a second disintegrant, a third disintegrant, a flavoring agent, and a lubricant.

In yet further embodiments, the spray-dried formulation contains fexofenadine zwitterionic dihydrate, a wetting agent, an alkalizing agent, a first buffer, a second buffer, a sweetener, a binder, a first diluent, a second diluent, a third diluent, a fourth diluent, a fifth diluent, a sixth diluent, a first disintegrant, a second disintegrant, a third disintegrant, a flavoring agent, and a lubricant.

In other embodiments, the spray-dried formulation contains fexofenadine zwitterionic dihydrate, a first wetting agent, a second wetting agent, an alkalizing agent, a first buffer, a second buffer, a sweetener, a binder, a first diluent, a second diluent, a third diluent, a first disintegrant, a second disintegrant, a third disintegrant, a flavoring agent, a first lubricant, and a second lubricant.

In further embodiments, the spray-dried formulation contains fexofenadine zwitterionic dihydrate, a wetting agent, an alkalizing agent, a first buffer, a second buffer, a sweetener, a binder, a first diluent, a second diluent, a third diluent, a first disintegrant, a second disintegrant, a third disintegrant, a flavoring agent, a first lubricant, and a second lubricant.

As described, the spray-dried formulation may also contain water. In some aspects, the water is sterile water.

The disclosure also provides oral solid dosage forms comprising a spray-dried formulation/granules or a final fexofenadine zwitterion composition as described herein. Examples of the oral solid dosage include tablets, caplets, or dispersible granules. In some aspects, the oral solid dosage form is a tablet such as an orally disintegrating tablet or a chewable tablet. In other aspects, the oral solid dosage form is a caplet, such as an orally disintegrating caplet or chewable caplet. In further aspects, the oral solid dosage form is dispersible granules. Desirably, the oral solid dosage form lacks a coating layer for bitter taste masking. In some embodiments, the oral dosage form lacks a coating layer that contains an Eudragit polymer. The term "chewable" as used herein refers to a dosage form that is intended to be chewed and then swallowed by the subject rather than swallowed whole. In some embodiments, the subject chews the chewable dosage form, thereby breaking the dosage form into finer particles having a higher surface area. Without wishing to be held to any particular theory, this higher surface area permits the fexofenadine zwitterion to be absorbed more quickly, as compared to an unchewed dosage form. In other embodiments, the dosage form in an orally disintegrating tablet that dissolves when it is held in the mouth of the subject. In further embodiments, the patient chews and dissolves the dosage form. In yet other embodiments, the patient swallows the dosage form.

The oral solid dosage form may contain an amount of fexofenadine zwitterion dihydrate that is determined by one skilled in the art. In some embodiments, the oral dosage form contains about 10 to about 200 mg of the fexofenadine zwitterion dihydrate. In some embodiments, the oral dosage form contains about 10, about 20, about 30, about 40, about 50, about 60, about 70, about 80, about 90, about 100, about 110, about 120, about 130, about 140, about 150, about 160, about 170, about 180, about 190, or about 200 mg of fexofenadine zwitterion dihydrate. In other embodiments, the oral dosage form contains about 10 to about 180, about 10 to about 160, about 10 to about 140, about 10 to about 120, about 10 to about 100, about 10 to about 80, about 10 to about 60, about 10 to about 40, about 20 to about 200, about 20 to about 180, about 20 to about 160, about 20 to about 140, about 20 to about 120, about 20 to about 100, about 20 to about 80, about 20 to about 60, about 20 to about 40, about 30 to about 200, about 30 to about 180, about 30 to about 160, about 30 to about 140, about 30 to about 120, about 30 to about 100, about 30 to about 80, about 30 to about 60, about 40 to about 200, about 40 to about 180, about 40 to about 160, about 40 to about 140, about 40 to about 120, about 40 to about 100, about 40 to about 80, about 40 to about 60, about 50 to about 200, about 50 to about 180, about 50 to about 160, about 50 to about 140, about 50 to about 120, about 50 to about 100, about 50 to about 80, about 60 to about 200, about 60 to about 180, about 60 to about 160, about 60 to about 140, about 60 to about 120, about 60 to about 100, about 60 to about 80, about 70 to about 200, about 70 to about 180, about 70 to about 160, about 70 to about 140, about 70 to about 120, about 70 to about 100, about 80 to about 200, about 80 to about 180, about 80 to about 160, about 80 to about 140, about 80 to about 120, about 80 to about 100, about 90 to about 200, about 90 to about 180, about 90 to about 160, about 90 to about 140, about 90 to about 120, about 100 to about 200, about 100 to about 180, about 100 to about 160, about 100 to about 140, about 100 to about 120, about 110 to about 200, about 110 to about 180, about 110 to about 160, about 110 to about 140, about 120 to about 200, about 120 to about 180, about 120 to about 160, about 120 to about 140, about 130 to about 200, about 130 to about 180, about 130 to about 160, about 140 to about 200, about 140 to about 180, about 140 to about 160, about 150 to about 200, about 150 to about 170, about 160 to about 200, about 160 to about 180, about 170 to about 200, about 180 to about 200, or about 190 to about 200. In further embodiments, the spray-dried formulation contains about 30 to about 180 mg of fexofenadine zwitterion dihydrate. In yet other embodiments, the spray-dried formulation contains about 30 mg of fexofenadine zwitterion dihydrate. In still further embodiments, the spray-dried formulation contains about 60 mg of the fexofenadine zwitterion dihydrate. In other embodiments, the spray-dried formulation contains about 120 mg of the fexofenadine zwitterion dihydrate. In further embodiments, the spray-dried formulation contains about 180 mg of the fexofenadine zwitterion dihydrate.

### Methods

The spray-dried compositions and oral solid dosage forms described herein are useful in relieving symptoms due to an allergy in a patient in need thereof. The methods (not claimed) include administering a therapeutically effective amount of the spray-dried formulation or oral solid dosage form to the patient.

One of skill in the would be able to determine suitable allergies that may be treated using the described compositions and dosage forms. For examples, the allergies may be due to indoor or outdoor allergens. In some embodiments, the allergy is due to one or more indoor allergens. A variety of indoor allergens are known and include dust mites, a pet allergen, or mold. In further embodiments, the allergy is due to dust mites. In other embodiments, the allergy is due to a pet allergen, e.g., such as in animal saliva, animal urine or animal dander. In still further embodiments, the allergy is due to indoor mold. In yet other embodiments, the allergy is due to one or more outdoor allergens. A number of outdoor allergies are known in the part and include, without limitation, pollen, and mold. In some embodiments, the outdoor allergy is pollen such as from grass, weeds, or trees. In other embodiments, the outdoor allergy is from outdoor mold.

The allergy may result in any number of symptoms in the patient. For examples, the patient may develop one or more of red eye, itchy eye, watery eye, itchy nose, runny nose, stuffy nose, sneezing, nasal congestion, wheezing, coughing, chest tightness, facial pain, rash, hives, shortness of breath, cough, postnasal drip, itchy throat, dry skin, sinus pressure, decreased sense of smell, decreased sense of taste, or poor sleep quality. In some embodiments, the symptom is red eye. In other embodiments, the symptom is itchy eye. In further embodiments, the symptom is watery eye. In yet other embodiments, the symptom is an itchy nose. In still further embodiments, the symptom is a runny nose. In other embodiments, the symptom is a stuffy nose. In further embodiments, the symptom is sneezing. In still other embodiments, the symptom is nasal congestion. In yet further embodiments, the symptom is wheezing. In other embodiments, the symptom is coughing. In further embodiments, the symptom is chest tightness. In yet other embodiments, the symptom is facial pain. In still further embodiments, the symptom is rash. In other embodiments, the symptom is hives. In further embodiments, the symptom is shortness of breath. In yet other embodiments, the symptom is a cough. In still further embodiments, the symptom is postnasal drip. In other embodiments, the symptom is an itchy throat. In further embodiments, the symptom is dry skin. In still other embodiments, the symptom is sinus pressure. In yet further embodiments, the symptom is decreased sense of smell. In other embodiments, the symptom is decreased sense of taste. In further embodiments, the symptom is poor sleep quality. Thus, the methods described herein are useful in treating these symptoms. In some embodiments, the methods ameliorate one or more, or all, of the symptoms. In other embodiments, the methods reduce the number of symptoms in the patient. In further embodiments, the methods prevent the onset of one or more symptoms in the patient.

The compositions and dosage forms are, thus, useful in relieving symptoms due to an upper respiratory allergy in a patient in need thereof. The methods include administering a therapeutically effective amount of the spray-dried formulation or oral solid dosage described herein to the patient. In some embodiments, the symptom is a runny nose, itchy, watery eye, sneezing, itching of the nose, itching of the throat, or a combination thereof. In further embodiments, the upper respiratory allergy is hay fever.

The following Examples are provided to illustrate some of the concepts described within this disclosure. While each Example is considered to provide specific individual embodiments of composition, methods of preparation and use, none of the Examples should be considered to limit the more general embodiments described herein.

In the following examples, efforts have been made to ensure accuracy with respect to numbers used (*e.g*., amounts, temperature, etc.) but some experimental error and deviation should be accounted for. Unless indicated otherwise, temperature is in degrees C, pressure is at or near atmospheric.

### Examples

### Example 1 - Preparation of the Fexofenadine Zwitterion Granules

Granules containing fexofenadine zwitterion granules were prepared as follows, using the components of Table 1.

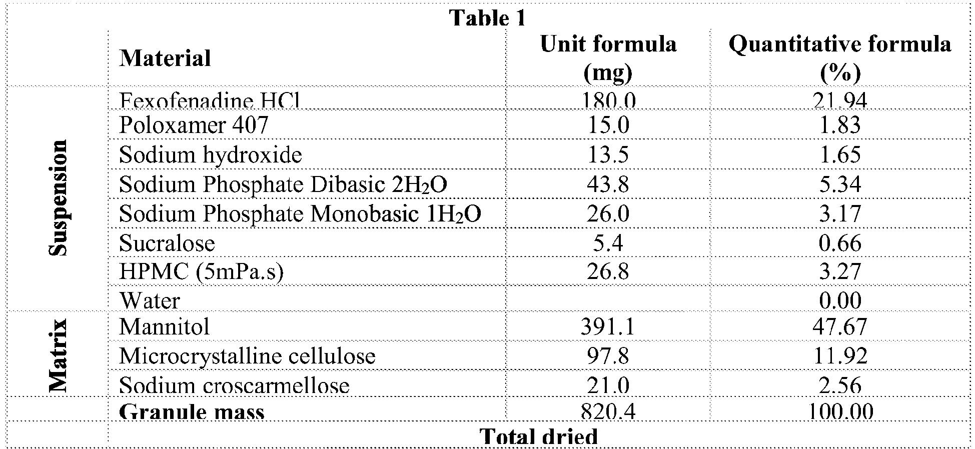

First, the fexofenadine zwitterion dihydrate suspension formulation is prepared at room temperature using a vessel having a rotor-stator.

Step 1: Poloxamer 407 is combined with water.

Step 2: Fexofenadine hydrochloride is incorporated into the mixture of water + poloxamer, *i.e.,* step 1, to form a suspension.

Step 3: Sodium hydroxide is then combined with the suspension using equimolar quantity of fexofenadine HCl to provide the fexofenadine zwitterion dihydrate and sodium chloride.

Step 4: A phosphate buffer, e.g., sodium phosphate dibasic dihydrate and sodium phosphate monobasic hydrate are added to the suspension of step 3 to maintain a pH in the range of 5.80 to about 7.0.

Step 5: Sucralose is added to the suspension.

Step 6: HPMC is included in the suspension to provide a final suspension having a pH around 6.7.

The final suspension was then sprayed and dried in an inert matrix to provide the granules. The spraying and drying was performed using a fluid bed and a tangential spray system. The inert matrix includes microcrystalline cellulose, mannitol, and sodium croscarmellose. After granulation, the granules were calibrated.

The final formulation was prepared by integrating an external phase with the granules. This was performed using starch glycolate, PEG6000, Poloxamer 407, a flavoring agent and magnesium stearate.

### Examples 2-26

The fexofenadine zwitterion dihydrate suspension and granules of Tables 2-26 were prepared as described in Example 1, but using the suspension and granule components noted in Tables 2-26. The granules of Tables 2-26 were then combined with the components of the external phase to provide the final fexofenadine zwitterion dihydrate formulation.

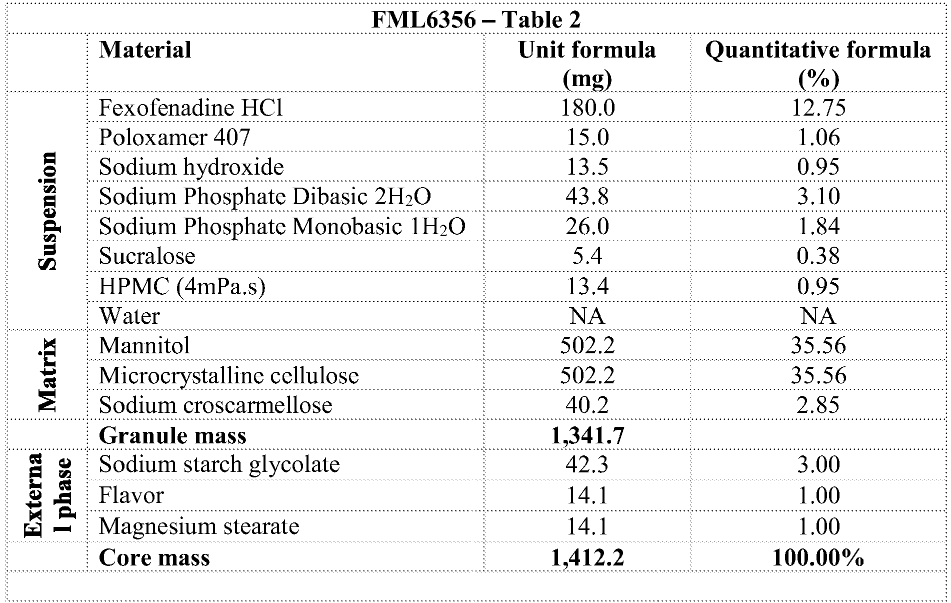

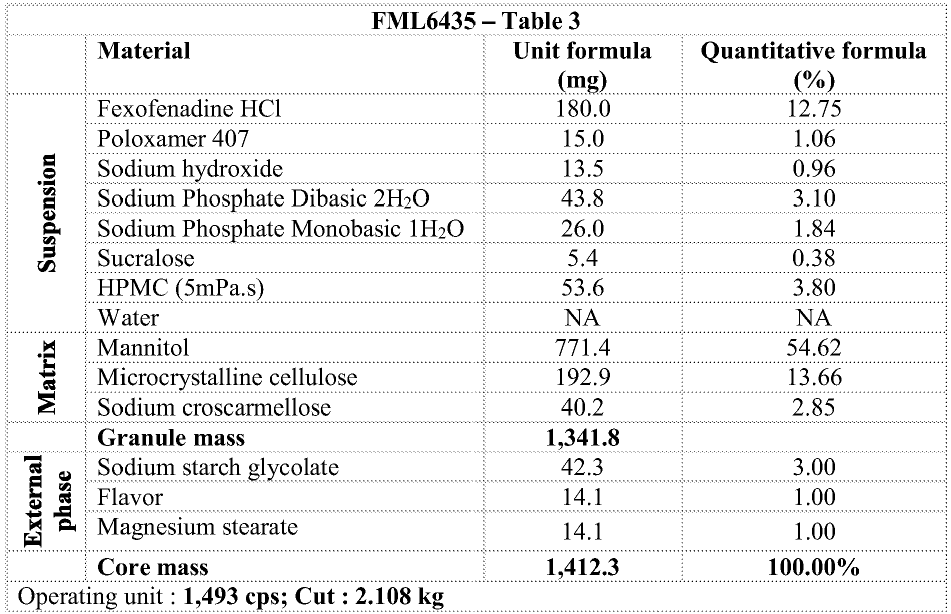

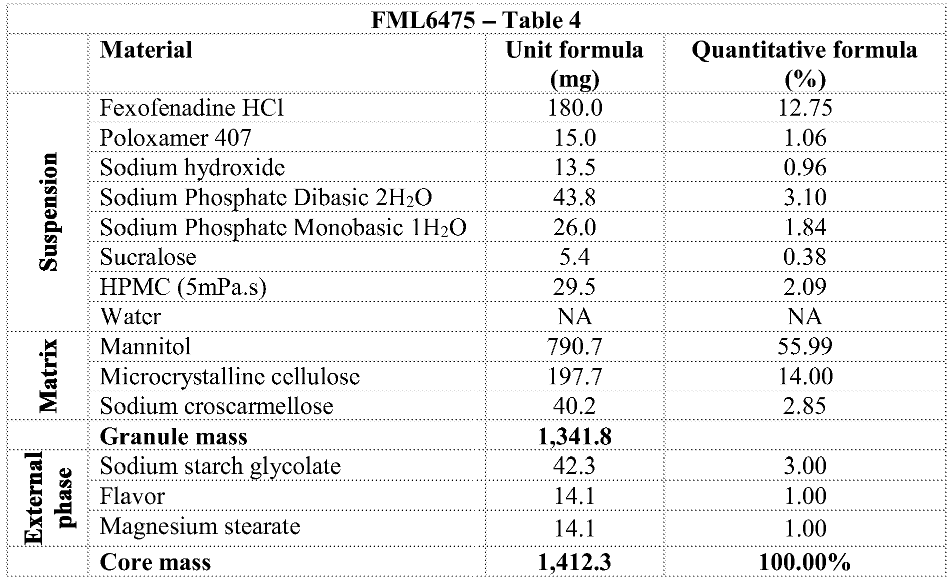

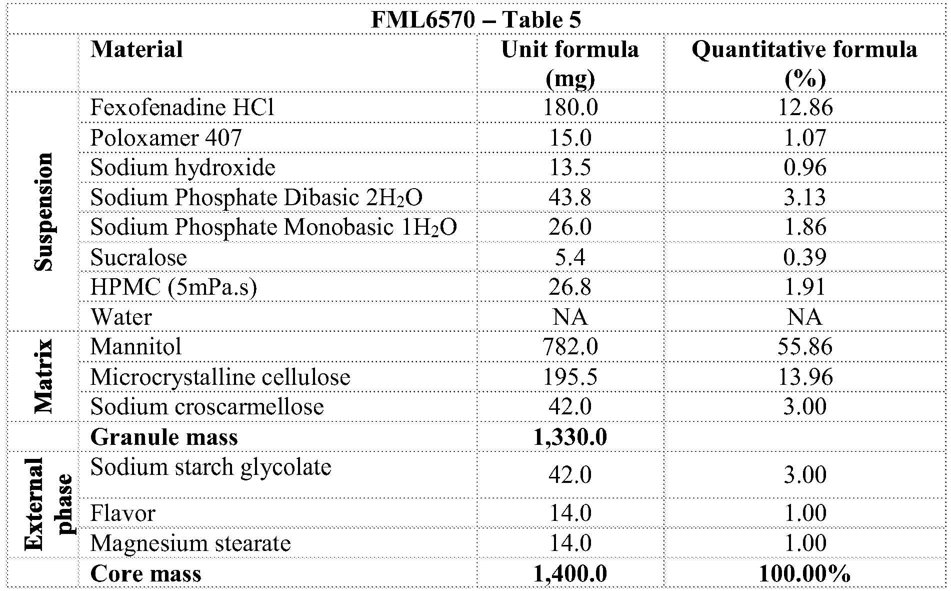

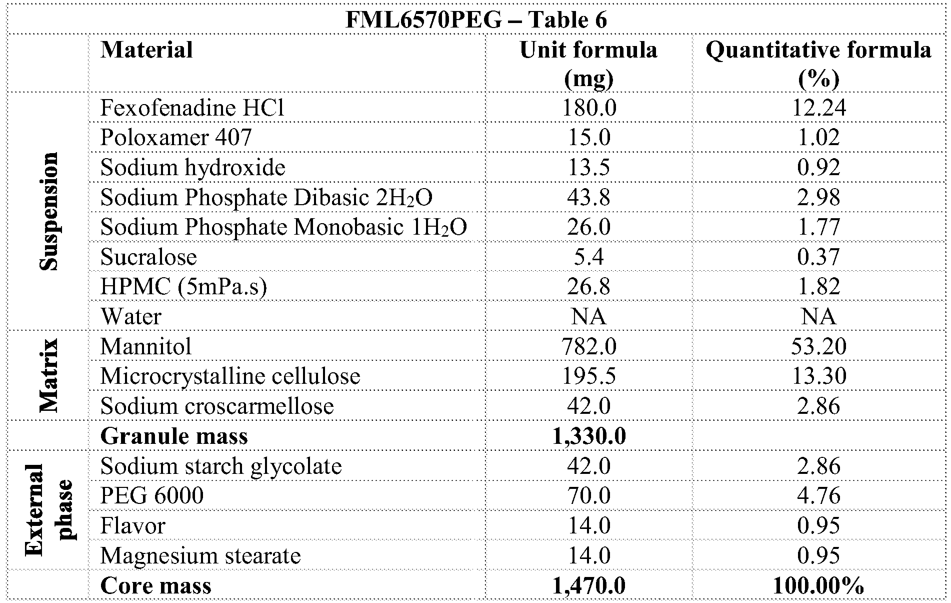

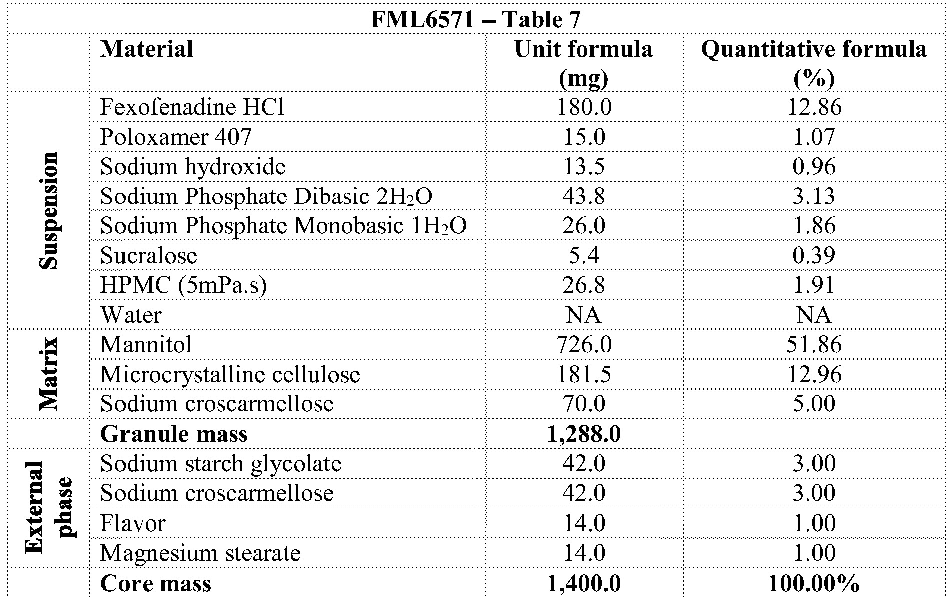

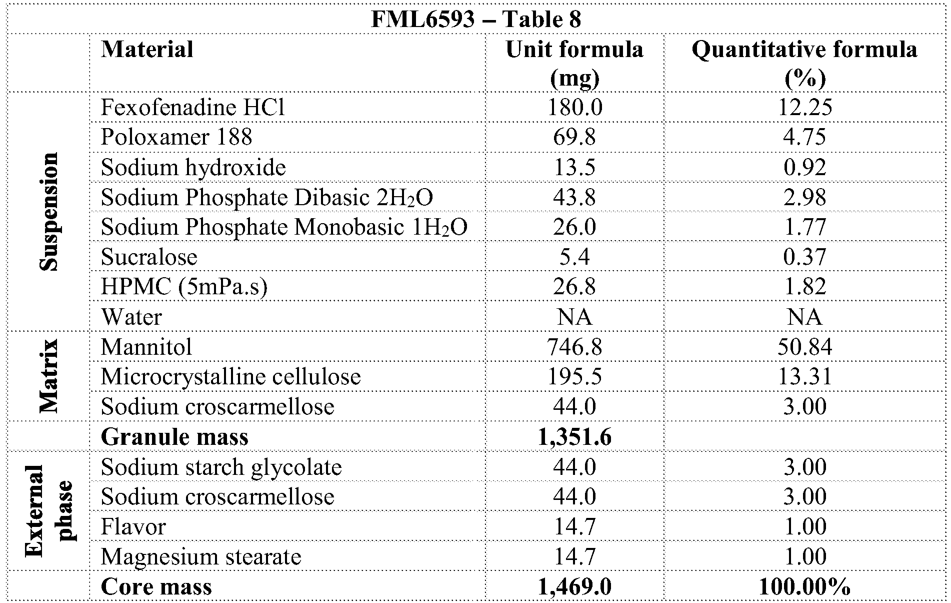

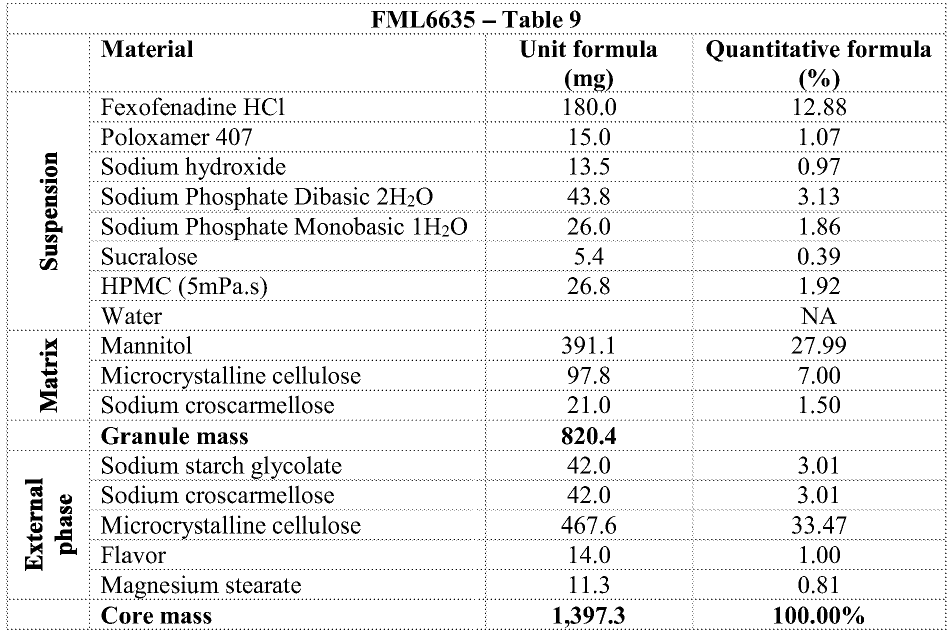

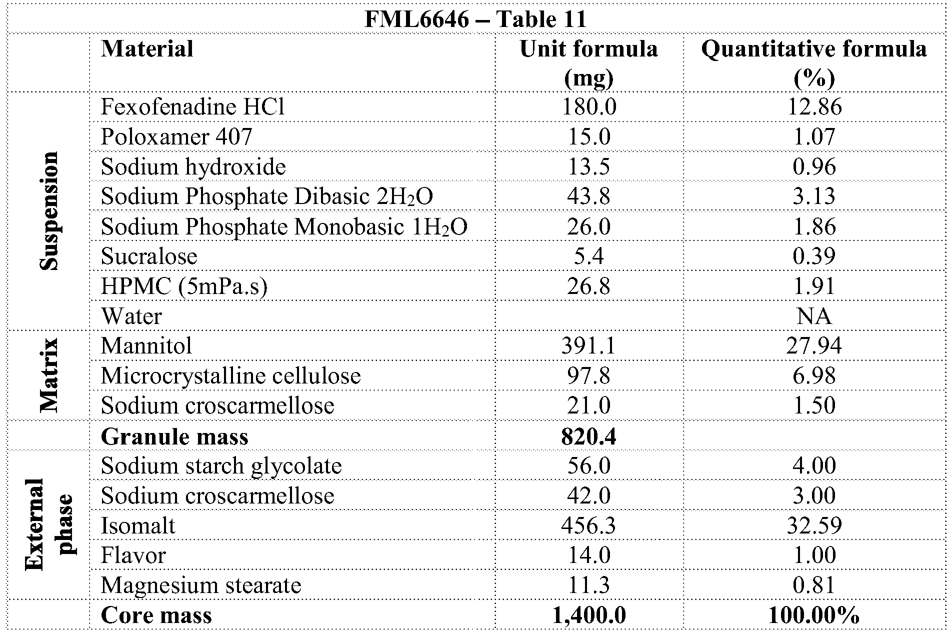

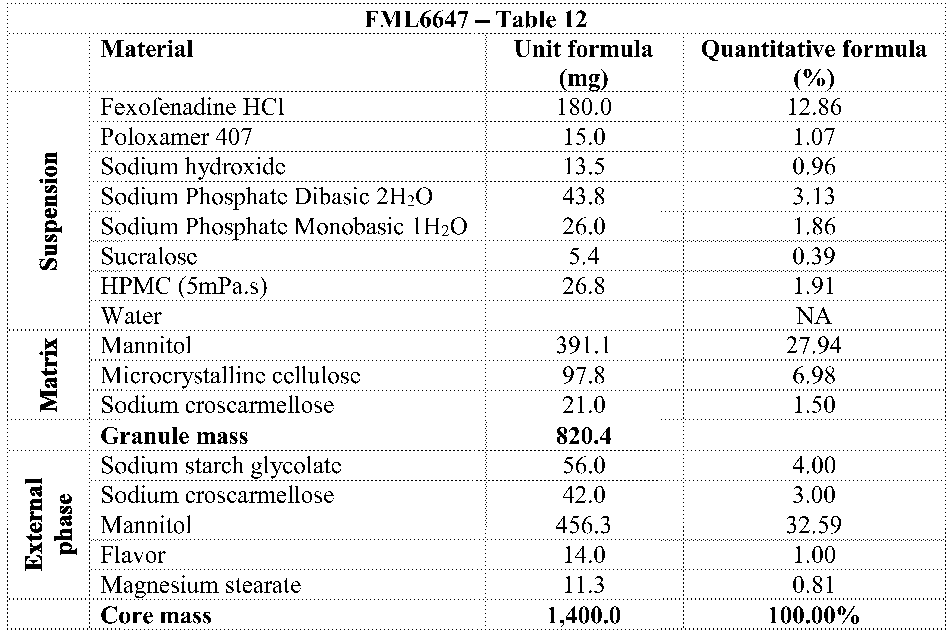

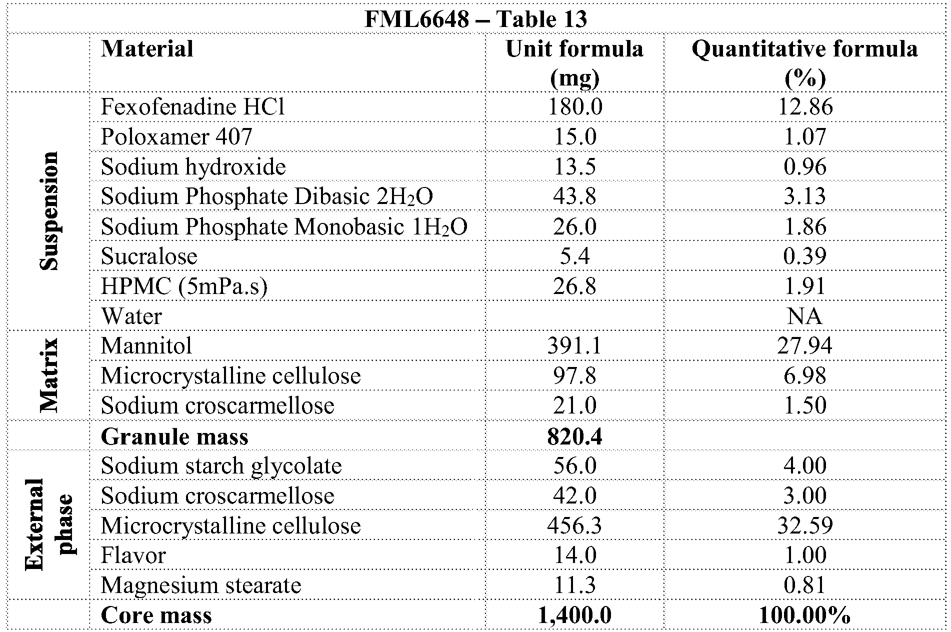

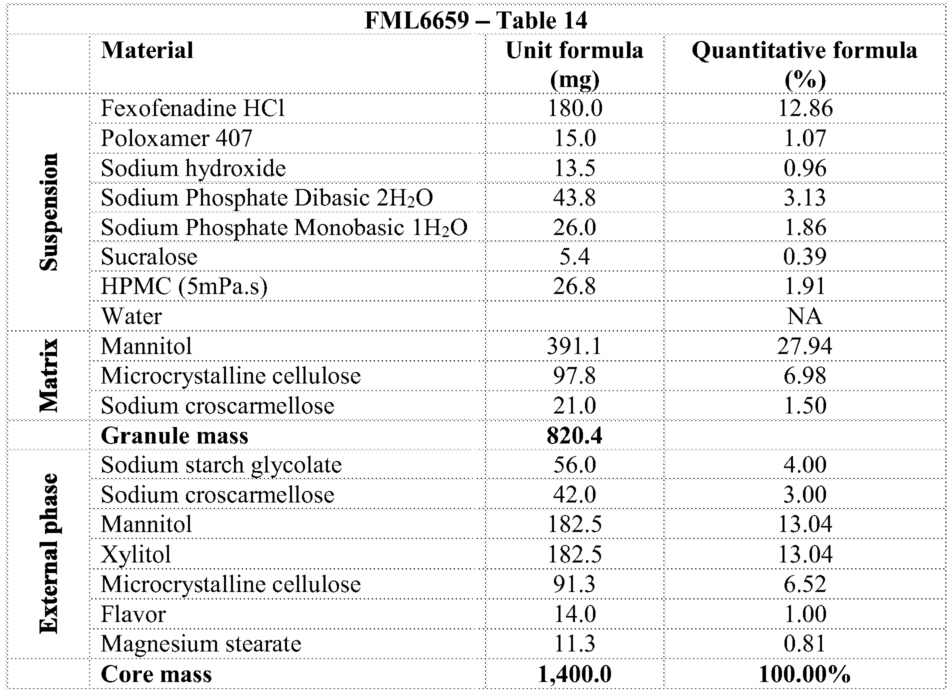

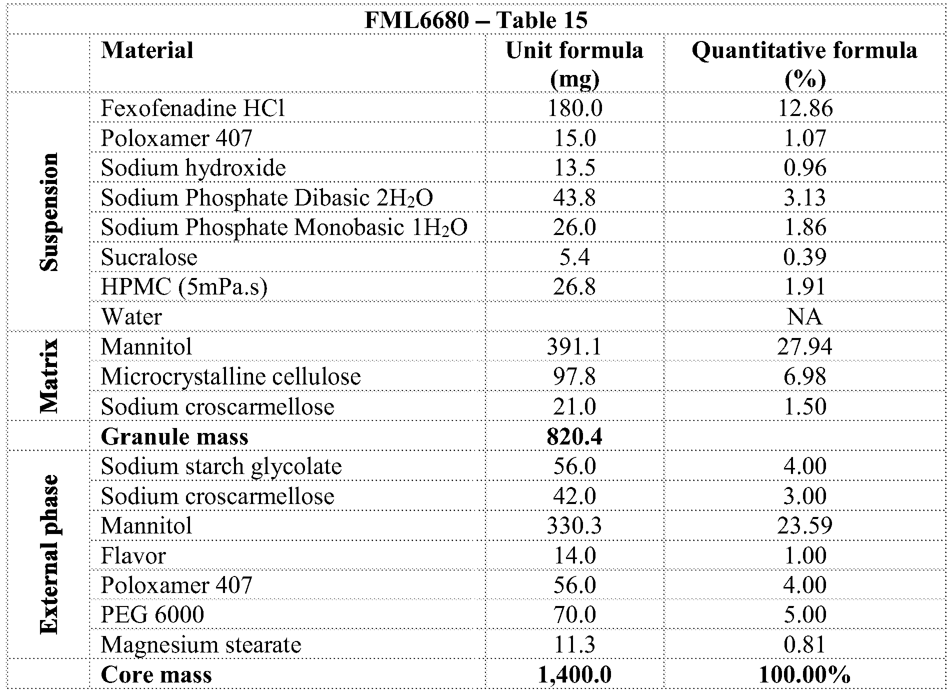

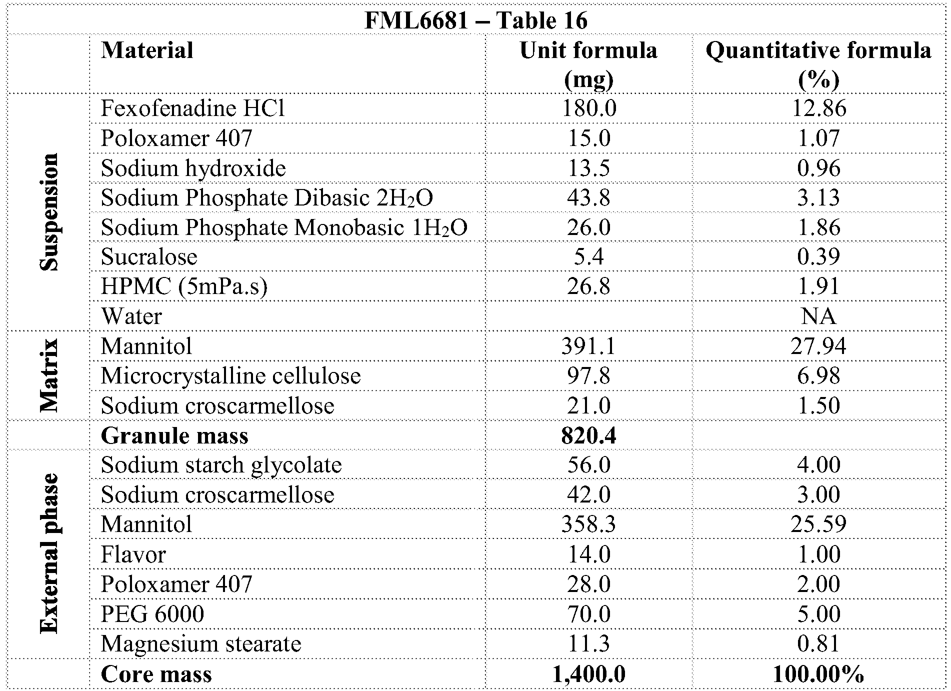

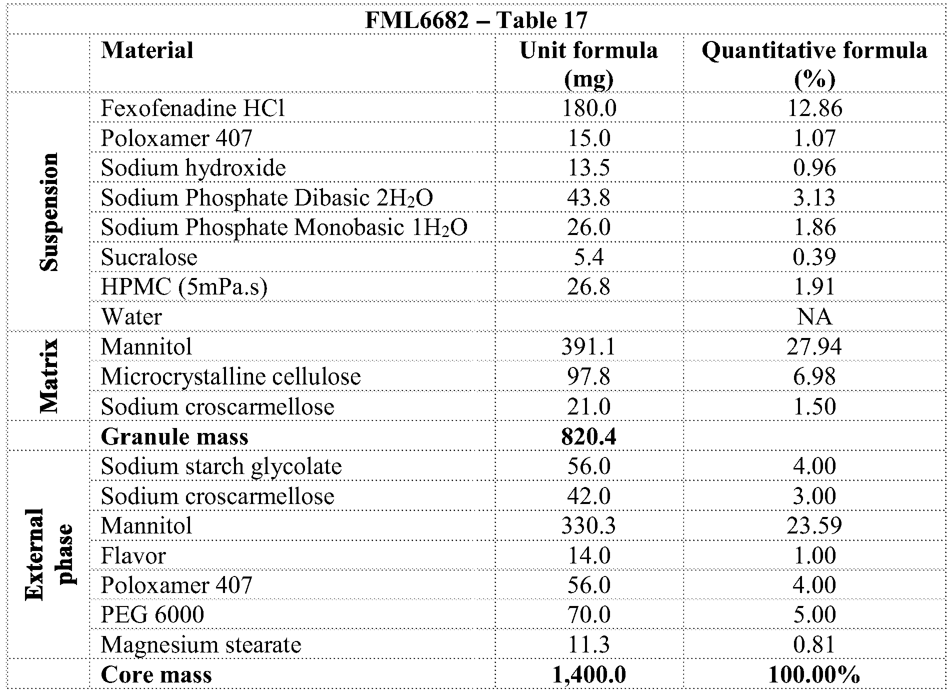

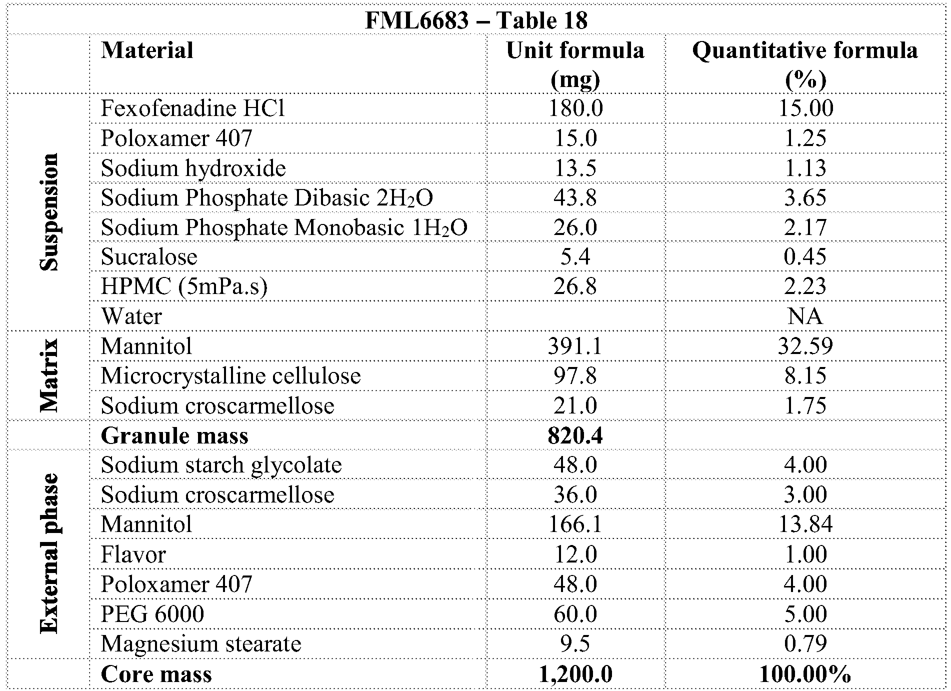

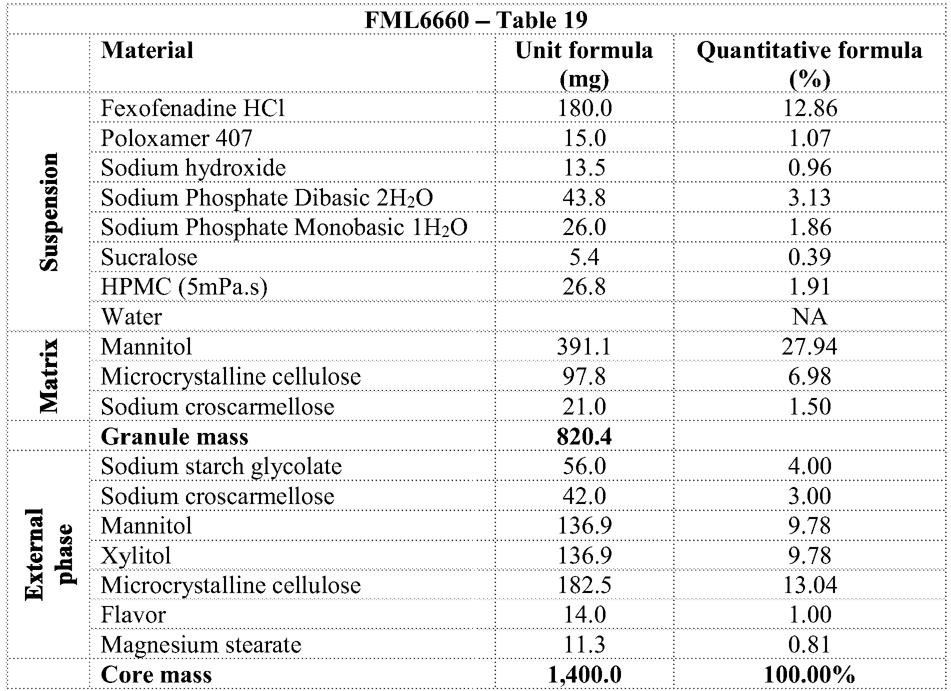

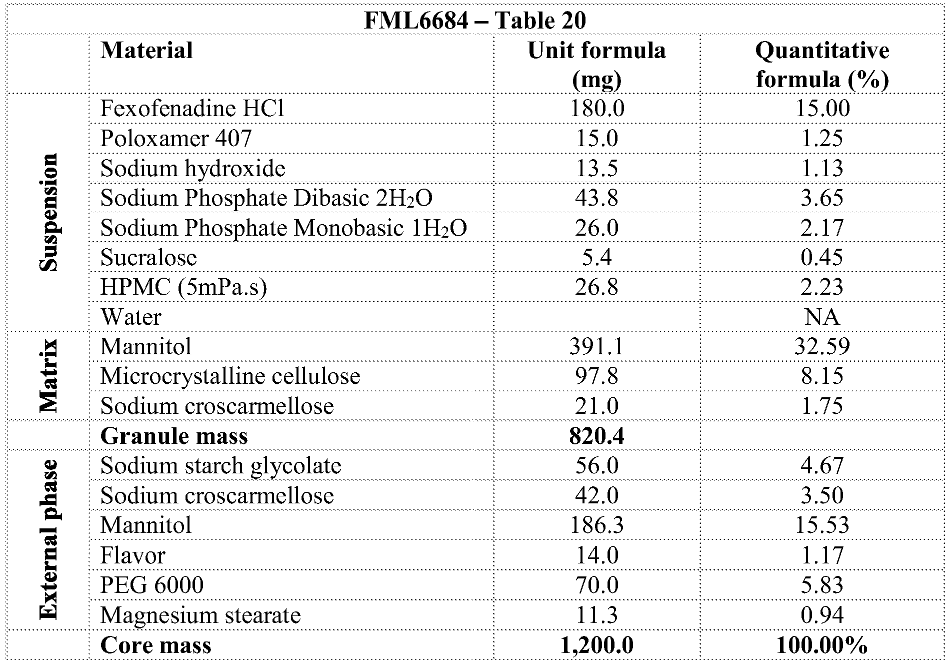

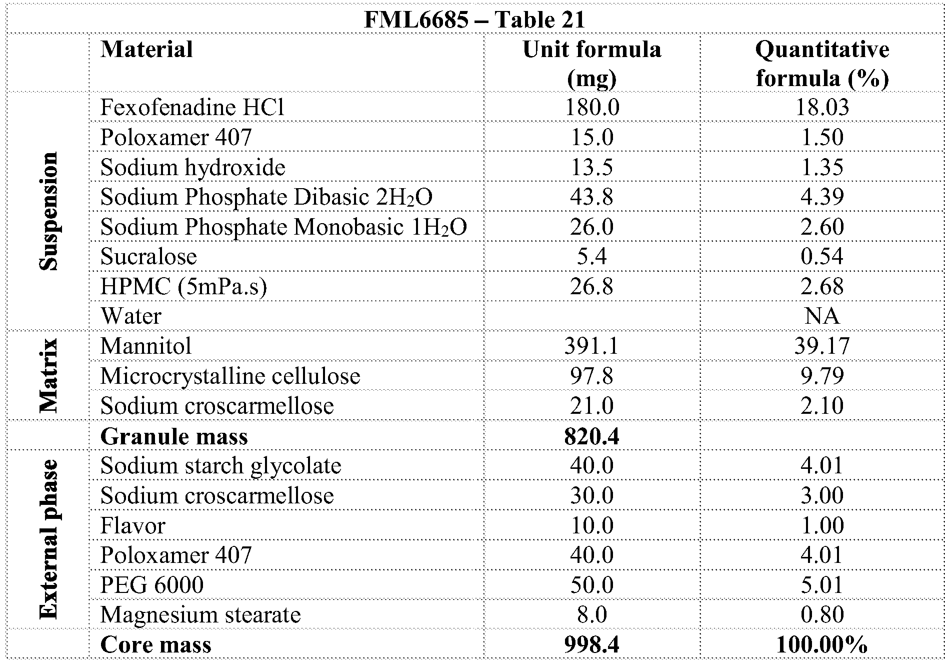

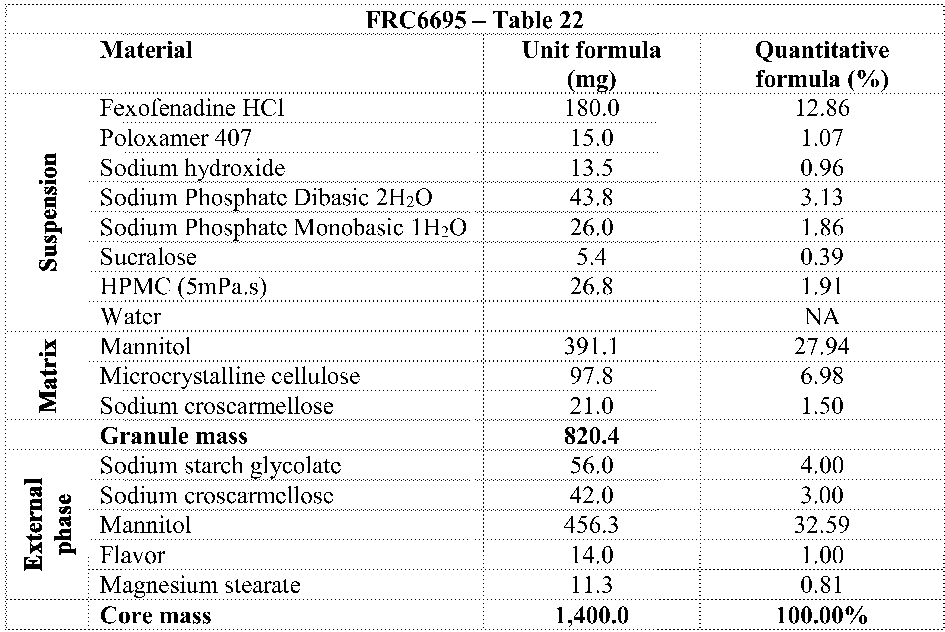

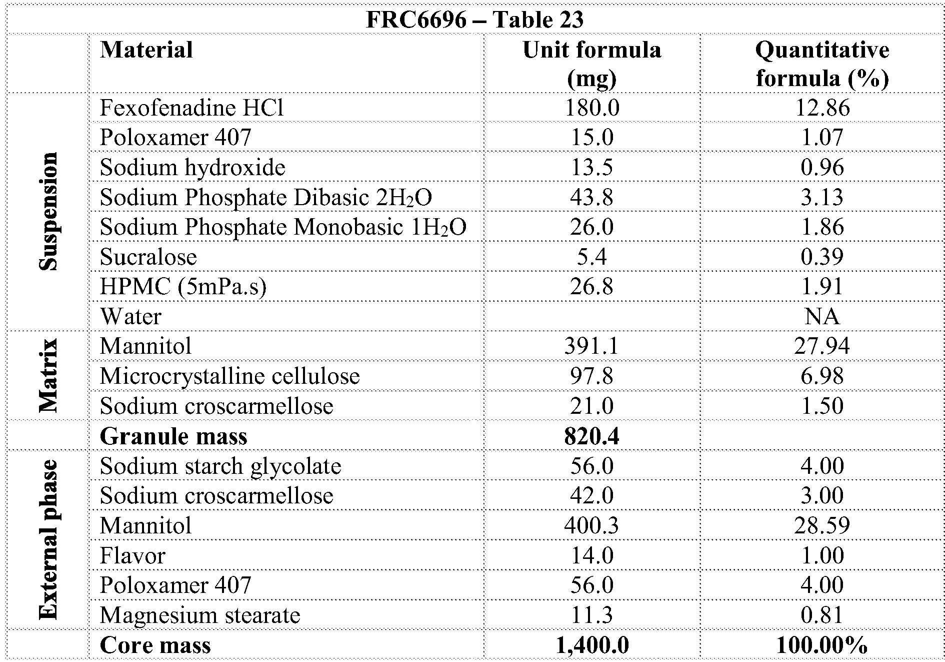

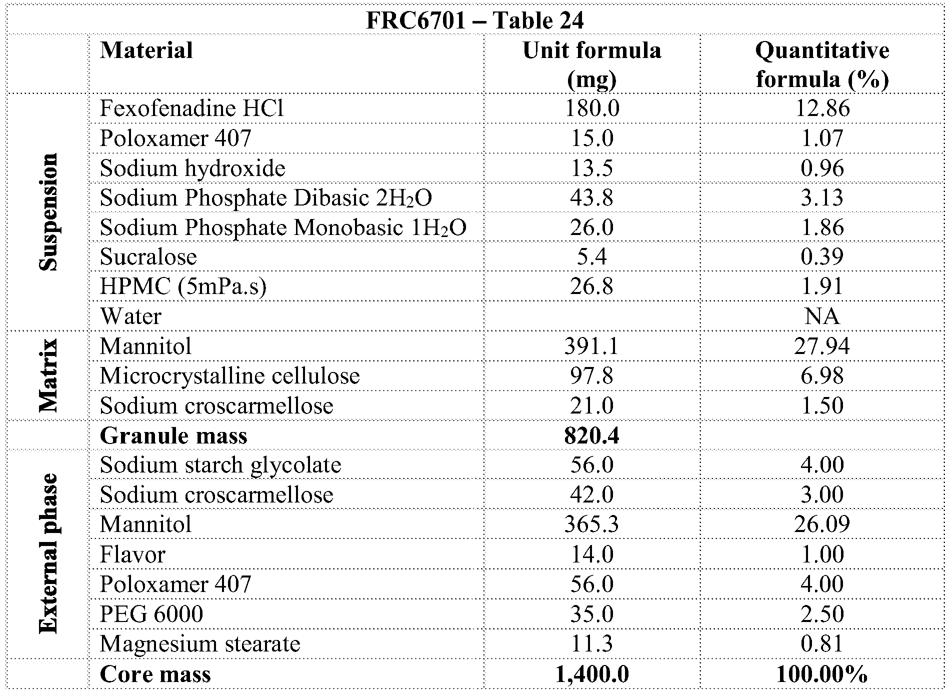

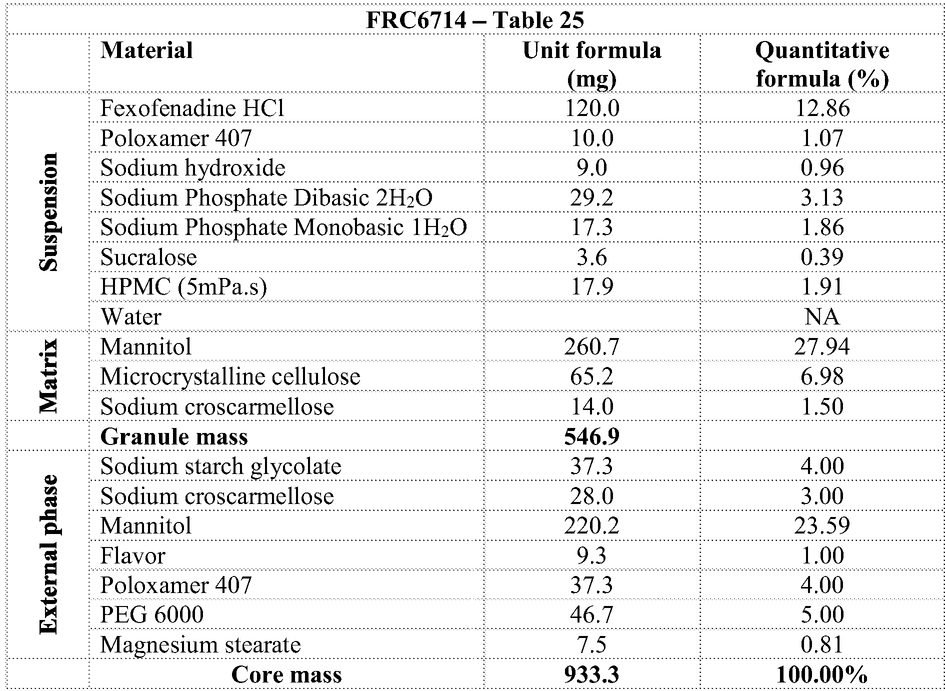

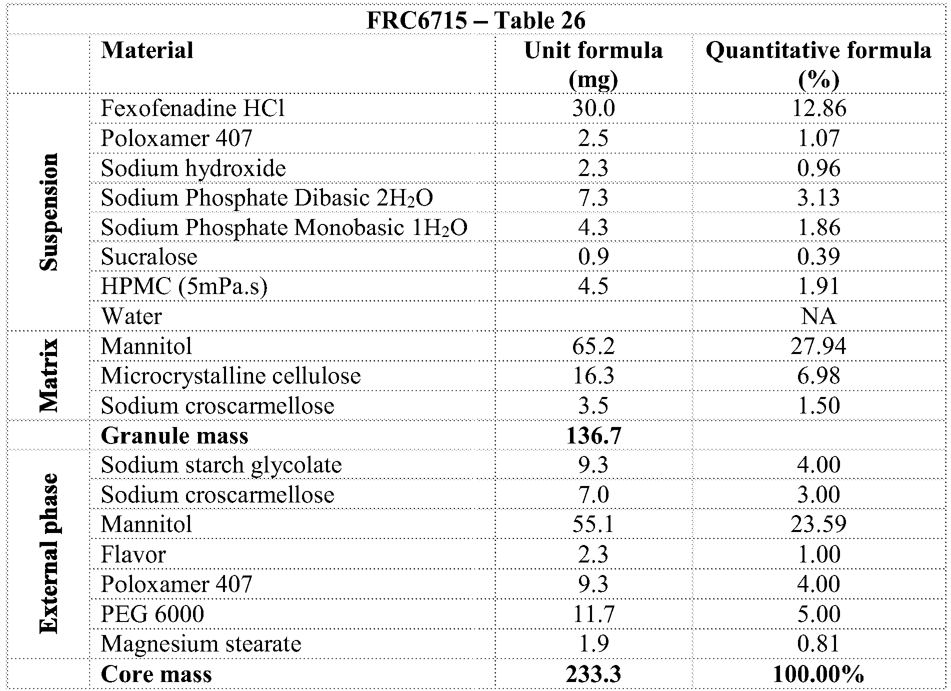

### Example 27: Bitterness Testing

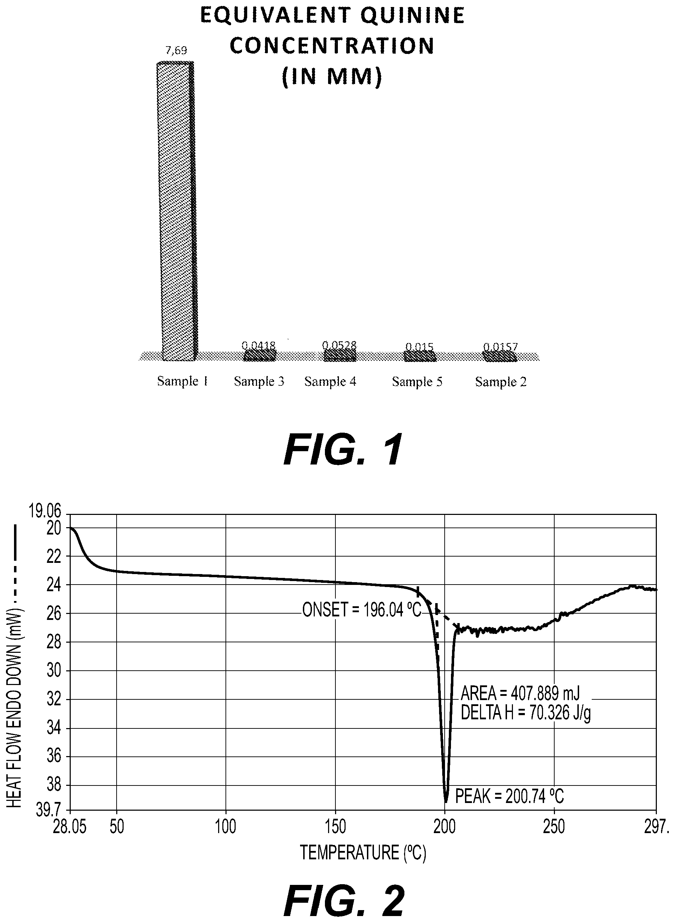

E-tongue analysis was carried out to compare the bitterness levels between samples of a suspension containing fexofenadine hydrochloride coated for taste masking (sample 1), fexofenadine zwitterion dihydrate of the disclosure (sample 2), a chewable tablet containing fexofenadine zwitterion dihydrate with grape flavor (sample 3), a chewable formulation containing fexofenadine zwitterion dihydrate chewable tablet with mint flavor (sample 4), and a chewable formulation containing spray-dried fexofenadine zwitterion dihydrate of the disclosure a grape flavor (sample 5). The results are presented in Figure 1. As a baseline, different concentrations of quinine, a bitter substance, were measured to determine the bitterness scale. Further, the different samples were measured, and the data generated were interpreted as bitterness levels of the quinine hydrochloride. The results show very low levels of bitterness for the fexofenadine zwitterion suspension of the disclosure as compared with compositions containing fexofenadine hydrochloride.

### Example 28: Characterization

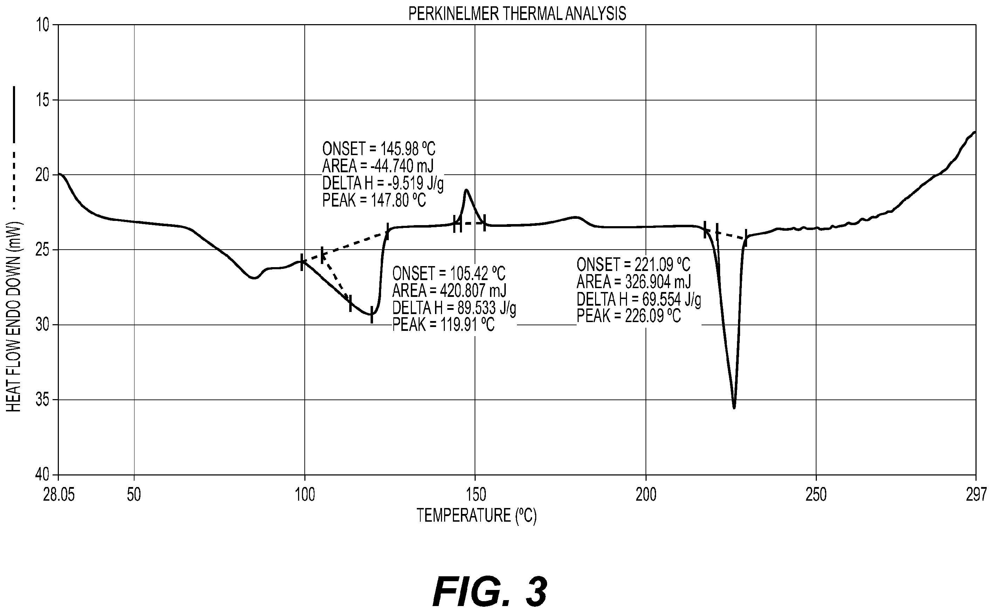

The fexofenadine zwitterion dihydrate prepared in Example 1 was characterized using differential scanning calorimetry (DSC) and compared to the DSC for fexofenadine hydrochloride. See, *e.g.,* Figs. 2 and 3. These results show that fexofenadine hydrochloride has a melting point at 196 - 200°C and fexofenadine zwitterion has a melting point of 220 - 230°C.

DSC analysis also was performed, comparing the DSC thermograms for fexofenadine zwitterion dihydrate and a spray-dried formulation of the disclosure containing fexofenadine zwitterion dihydrate. The results showed that the DSC for spray-dried formulation of the disclosure containing fexofenadine zwitterion dihydrate showed a thermal event at the same range as the melting point for fexofenadine zwitterion.

## Claims

1. A process of preparing a spray-dried formulation comprising fexofenadine zwitterion dihydrate comprising spray-drying (i) a fexofenadine zwitterion dihydrate composition; and (ii) an inert matrix.

2. The process of claim 1, wherein the inert matrix comprises one or more pharmaceutically acceptable excipients such as a diluent, binder, glidant, or disintegrant, or a combination thereof.

3. The process of claim 2, wherein:
the diluent is microcrystalline cellulose, mannitol, maltitol, sucrose, erythritol, calcium phosphate dibasic, calcium phosphate tribasic, isomalt, xylitol, maltodextrin, lactose, starch, sorbitol, sucrose, a sucrose derivative, invert sucrose, lactitol, erythritol, fructose, glucose, calcium carbonate, cellulose, a cellulose derivative, calcium carbonate, calcium lactate, calcium sulfate, or calcium phosphate, or a combination thereof; or
the disintegrant is sodium croscarmellose, sodium starch glycolate, crospovidone, starch pregelatinized, starch, or hydroxypropylcellulose (HPC), or a combination thereof.

4. The process of any one of claims 1-3, further comprising:
(a) combining fexofenadine hydrochloride, water, and wetting agent;
(b) combining an alkalizing agent with the product of step (a);
(c) adjusting the pH of the product of step (b) to a pH of 5.8 to 7 using one or more of a buffer;
(d) combining a sweetener with the product of step (c); and
(e) combining the product of step (d) with a binder;
wherein the fexofenadine zwitterion dihydrate composition has a pH of 6.5 to 7, preferably 6.7.

5. The process of claim 4, further comprising combining the spray-dried formulation with one or more of a second portion of the disintegrant, a second disintegrant, a second portion of the diluent, a flavoring agent, a second portion of the wetting agent, and one or more lubricants.

6. The process of claim 5, wherein:
the second disintegrant is sodium starch glycolate, crospovidone, starch pregelatinized, starch, hydroxypropylcellulose (HPC), or a combination thereof; or
the lubricant is polyethylene glycol (PEG) such as PEG 6000, magnesium stearate, sodium stearyl fumarate, stearic acid, sorbitan monostearate, sucrose monopalmitate, glyceryl monostearate, glyceryl tribehenate, glyceryl dibehenate, calcium stearate, and zinc stearate, hydrated magnesium silicate, sodium oleate, edible hydrogenated vegetable oil, sodium benzoate, talc, sodium acetate, a wax, sodium lauryl sulfate, or magnesium lauryl sulfate, or a combination thereof.

7. A product prepared according to the process of any one of the preceding claims.

8. A spray-dried formulation comprising fexofenadine zwitterion dihydrate and an inert matrix.

9. The spray-dried formulation of claim 8, wherein the inert matrix comprises one or more pharmaceutically acceptable excipients, such as a diluent or a first disintegrant.

10. The spray-dried formulation of claim 9, wherein:
the diluent is microcrystalline cellulose, mannitol, isomalt, xylitol, or a combination thereof, preferably microcrystalline cellulose and mannitol, preferably microcrystalline cellulose, mannitol, maltitol, maltodextrin, lactose, starch, sorbitol, sucrose, a sucrose derivative, invert sucrose, lactitol, erythritol, fructose, glucose, calcium carbonate, cellulose, a cellulose derivative, calcium carbonate, calcium lactate, calcium sulfate, or calcium phosphate, or a combination thereof; or
the first disintegrant is sodium croscarmellose, sodium starch glycolate, crospovidone, starch pregelatinized, starch, hydroxypropylcellulose (HPC), or a combination thereof.

11. The spray-dried formulation of any one of claims 8-10, wherein the formulation prior to spray-drying further comprises one or more of a wetting agent, alkalizing agent, buffer, sweetener, binder, or solvent, optionally further comprising one or more of a second disintegrant, flavoring agent, and lubricant.

12. The spray-dried formulation of claim 11, wherein:
the wetting agent comprises a poloxamer, such as poloxamer 188, poloxamer 237, poloxamer 338, poloxamer 407, or a combination thereof; or
the alkalizing agent is a strong base such as potassium hydroxide, calcium hydroxide, sodium hydroxide, or barium hydroxide; or
the buffer is sodium phosphate dibasic 2H₂O, sodium phosphate monobasic, citric acid/sodium phosphate dibasic, sodium phosphate dibasic hydrate, succinic acid/sodium hydroxide, citric acid/sodium citrate, sodium citrate hydrate, potassium citrate, maleic acid/sodium hydroxide, fumaric acid/sodium hydroxide, sodium phosphate monobasic hydrate, potassium phosphate monobasic/sodium phosphate dibasic, sodium phosphate dibasic hydrate, potassium phosphate dibasic, potassium phosphate dibasic hydrate, or preferably sodium phosphate monobasic, sodium phosphate monobasic hydrate, potassium phosphate monobasic/sodium phosphate dibasic, sodium phosphate dibasic hydrate, potassium phosphate dibasic, or potassium phosphate dibasic hydrate, or more preferably sodium phosphate monobasic, sodium phosphate monobasic hydrate/sodium phosphate dibasic, or sodium phosphate dibasic hydrate, or a combination thereof, or preferably phosphate dibasic and phosphate monobasic; or
the sweetener is sucralose, aspartame, acesulfame potassium, saccharin, steviol glycosides, neotame, advantame, saccharin sodium, cyclamate sodium, or ammonium glycyrrhizate, or a combination thereof; or
the binder is hydroxypropylmethylcellulose (HPMC), such as HPMC 4mPa.s or HPMC 5 mPa.s., hydroxypropylcellulose (HPC), polyvinylpyrrolidone (PVP), or carboxymethyl cellulose (CMC), hydroxyethylmethyl cellulose (HEMC), or hydroxyethylcellulose (HEC), or a combination thereof; or
the second disintegrant is sodium starch glycolate, crospovidone, starch pregelatinized, starch, or hydroxypropylcellulose (HPC), or a combination thereof; or
the lubricant is polyethylene glycol, such as polyethylene glycol 6000, magnesium stearate, crospovidone, starch pregelatinized, starch, or hydroxypropylcellulose (HPC), or a combination thereof.

13. The spray-dried formulation of any one of claims 8-12, wherein upon contact with a human subject's tongue, does not produce a bitter taste.

14. A spray-dried formulation comprising:
fexofenadine zwitterion dihydrate;
a wetting agent;
an alkalizing agent;
a buffer;
a sweetener;
a binder;
a diluent;
a disintegrant;
a flavoring agent; and
a lubricant.

15. The spray-dried formulation of claim 14, wherein:
the wetting agent comprises a poloxamer, such as poloxamer 188, poloxamer 407, poloxamer 237, poloxamer 338, a polysorbate such as polysorbate 80 or a mixture of polysorbate 80 and magnesium aluminometasilicate, or a polyoxyl hydrogenated castor oil, such as a mixture of polyoxyl 40 hydrogenated castor oil and magnesium aluminosilicate, or a combination thereof; or
the alkalizing agent is a strong base such as potassium hydroxide, calcium hydroxide, sodium hydroxide, or barium hydroxide; or
the buffer is sodium phosphate dibasic 2H₂O, sodium phosphate monobasic, citric acid/sodium phosphate dibasic, sodium phosphate dibasic hydrate, succinic acid/sodium hydroxide, citric acid/sodium citrate, sodium citrate hydrate, potassium citrate, maleic acid/sodium hydroxide, fumaric acid/sodium hydroxide, sodium phosphate monobasic hydrate, potassium phosphate monobasic/sodium phosphate dibasic, sodium phosphate dibasic hydrate, potassium phosphate dibasic, potassium phosphate dibasic hydrate, or preferably sodium phosphate monobasic, sodium phosphate monobasic hydrate, potassium phosphate monobasic/sodium phosphate dibasic, sodium phosphate dibasic hydrate, potassium phosphate dibasic, or potassium phosphate dibasic hydrate, or more preferably sodium phosphate monobasic, sodium phosphate monobasic hydrate/sodium phosphate dibasic, or sodium phosphate dibasic hydrate, or a combination thereof, or preferably phosphate dibasic and phosphate monobasic; or
the sweetener is sucralose, aspartame, acesulfame potassium, saccharin, steviol glycoside, neotame, advantame, saccharin sodium, cyclamate sodium, or ammonium glycyrrhizate, or a combination thereof; or
the binder is hydroxypropylmethylcellulose (HPMC), such as HPMC 4mPa.s or HPMC 5 mPa.s, hydroxypropylcellulose (HPC), polyvinylpyrrolidone (PVP), or carboxymethyl cellulose (CMC), hydroxyethylmethyl cellulose (HEMC), or hydroxyethylcellulose (HEC), or a combination thereof; or
the diluent is microcrystalline cellulose, mannitol, isomalt, xylitol, maltitol, maltodextrin, lactose, starch, sorbitol, sucrose, a sucrose derivative, invert sucrose, lactitol, erythritol, fructose, glucose, calcium carbonate, cellulose, a cellulose derivative, calcium carbonate, calcium lactate, calcium sulfate, or calcium phosphate, or a combination thereof, preferably microcrystalline cellulose and mannitol; or
the disintegrant is sodium croscarmellose, sodium starch glycolate, sodium starch glycolate, crospovidone, starch pregelatinized, starch, or hydroxypropylcellulose (HPC), or a combination thereof, or a combination thereof; or
the lubricant is polyethylene glycol, such as polyethylene glycol 6000, magnesium stearate, sodium stearyl fumarate, stearic acid, sorbitan monostearate, sucrose monopalmitate, glyceryl monostearate, glyceryl tribehenate, glyceryl dibehenate, calcium stearate, and zinc stearate, hydrated magnesium silicate, sodium oleate, edible hydrogenated vegetable oil, sodium benzoate, talc, sodium acetate, a wax, sodium lauryl sulfate, or magnesium lauryl sulfate, or a combination thereof.

16. The spray-dried formulation of claim 14 or 15, comprising:
about 0.5 to about 10% w/w of the wetting agent; or
about 0.5 to about 5% w/w of the alkalizing agent; or
about 0.2 to about 10% w/w, of the buffer; or
about 0.2 to about 3% w/w of the sweetener; or
about 0.5 to about 5% w/w of the binder; or
about 30 to about 80% w/w of the diluent; or
about 3 to about 15% w/w of the disintegrant; or
about 3 to about 15% w/w of the disintegrant; or
about 0.5 to about 10% w/w of the lubricant; or
about 0.1 to about 5% w/w of the flavoring agent.

17. An oral solid dosage form comprising the spray-dried formulation of any one of claims 14-16, optionally lacking a coating layer for bitter taste masking.

18. The oral solid dosage form of claim 17, that is solid dosage form, such as a tablet, *e.g.,* orally disintegrating tablet, chewable tablet, or dispersible granules, or a liquid dosage form, such as a suspension, preferably a chewable tablet, optionally comprising about 10 to about 200 mg of fexofenadine zwitterion, such as about 30 to about 180 mg, or such as about 30 mg, about 60 mg, about 120 mg, or about 180 mg.

19. The spray-dried formulation of any one of claims 8-16 or the oral solid dosage form of claim 17 or 18 for relieving symptoms due to an allergy such as an upper respiratory allergy in a patient in need thereof.

20. The spray-dried formulation or oral solid dosage form of claim 19, wherein the allergy is due to an indoor allergen such as dust mites, a pet allergen such as in animal saliva, animal urine or animal dander, or mold or outdoor allergen such as pollen such as from grass, weeds, or trees, such as hay fever, or mold.

## Patentansprüche

1. Prozess zur Herstellung einer sprühgetrockneten Formulierung, die Fexofenadin-Zwitterion-Dihydrat umfasst, umfassend Sprühtrocknen (i) einer Fexofenadin-Zwitterion-Dihydrat-Zusammensetzung und (ii) einer inerten Matrix.

2. Prozess nach Anspruch 1, wobei die inerte Matrix einen oder mehrere pharmazeutisch akzeptable Hilfsstoffe wie etwa ein Verdünnungsmittel, Bindemittel, Gleitmittel oder Sprengmittel oder eine Kombination davon umfasst.

3. Prozess nach Anspruch 2, wobei:
das Verdünnungsmittel mikrokristalline Cellulose, Mannitol, Maltitol, Saccharose, Erythrit, Dicalciumphosphat, Tricalciumphosphat, Isomalt, Xylit, Maltodextrin, Lactose, Stärke, Sorbitol, Saccharose, ein Saccharosederivat, Invertzucker, lactitol, Erythrit, Fructose, Glucose, Calciumcarbonat, Cellulose, ein Cellulosederivat, Calciumcarbonat, Calciumlactat, Calciumsulfat oder Calciumphosphat oder eine Kombination davon ist; oder
das Sprengmittel Natriumcroscarmellose, Natriumstärkeglykolat, Crospovidon, vorverkleisterte Stärke, Stärke oder Hydroxypropylcellulose (HPC) oder eine Kombination davon ist.

4. Prozess nach einem der Ansprüche 1-3, weiter umfassend:
(a) Kombinieren von Fexofenadinhydrochlorid, Wasser und Netzmittel;
b) Kombinieren eines Alkalisierungsmittels mit dem Produkt aus Schritt (a);
(c) Einstellen des pH-Werts des Produkts aus Schritt (b) auf einen pH-Wert von 5,8 bis 7 unter Verwendung eines oder mehrerer Puffer;
(d) Kombinieren eines Süßstoffs mit dem Produkt aus Schritt (c); und
(e) Kombinieren des Produkts aus Schritt (d) mit einem Bindemittel;
wobei die Fexofenadin-Zwitterion-Dihydrat-Zusammensetzung einen pH-Wert von 6,5 bis 7, vorzugsweise von 6,7 aufweist.

5. Prozess nach Anspruch 4, weiter umfassend Kombinieren der sprühgetrockneten Formulierung mit einem oder mehreren einer zweiten Portion des Sprengmittels, eines zweiten Sprengmittels, einer zweiten Portion des Verdünnungsmittels, eines Aromastoffs, einer zweiten Portion des Netzmittels und eines oder mehrerer Schmiermittel.

6. Prozess nach Anspruch 5, wobei:
das zweite Sprengmittel Natriumstärkeglykolat, Crospovidon, vorverkleisterte Stärke, Stärke, Hydroxypropylcellulose (HPC) oder eine Kombination davon ist; oder
das Schmiermittel Polyethylenglykol (PEG), wie PEG 6000, Magnesiumstearat, Natriumstearylfumarat, Stearinsäure, Sorbitanmonostearat, Saccharosemonopalmitat, Glycerylmonostearat, Glyceryltribehenat, Glyceryldibehenat, Calciumstearat und Zinkstearat, hydratisiertes Magnesiumsilikat, Natriumoleat, essbares hydriertes Pflanzenöl, Natriumbenzoat, Talkum, Natriumacetat, ein Wachs, Natriumlaurylsulfat oder Magnesiumlaurylsulfat oder eine Kombination davon ist.

7. Produkt, das nach dem Prozess nach einem der vorstehenden Ansprüche hergestellt wurde.

8. Sprühgetrocknete Formulierung, umfassend Fexofenadin-Zwitterion-Dihydrat und eine inerte Matrix.

9. Sprühgetrocknete Formulierung nach Anspruch 8, wobei die inerte Matrix einen oder mehrere pharmazeutisch akzeptable Hilfsstoffe, wie etwa ein Verdünnungsmittel oder ein erstes Sprengmittel, umfasst.

10. Sprühgetrocknete Formulierung nach Anspruch 9, wobei:
das Verdünnungsmittel mikrokristalline Cellulose, Mannitol, Isomalt, Xylit oder eine Kombination davon, vorzugsweise mikrokristalline Cellulose und Mannitol, vorzugsweise mikrokristalline Cellulose, Mannitol, Maltitol, Maltodextrin, Lactose, Stärke, Sorbitol, Saccharose, ein Saccharosederivat, Invertzucker, lactitol, Erythrit, Fructose, Glucose, Calciumcarbonat, Cellulose, ein Cellulosederivat, Calciumcarbonat, Calciumlactat, Calciumsulfat oder Calciumphosphat oder eine Kombination davon ist; oder
das erste Sprengmittel Natriumcroscarmellose, Natriumstärkeglykolat, Crospovidon, vorverkleisterte Stärke, Stärke, Hydroxypropylcellulose (HPC) oder eine Kombination davon ist.

11. Sprühgetrocknete Formulierung nach einem der Ansprüche 8-10, wobei die Formulierung vor dem Sprühtrocknen weiter eines oder mehrere von einem Benetzungsmittel, Alkalisierungsmittel, Puffer, Süßstoff, Bindemittel oder Lösungsmittel umfasst, optional weiter eines oder mehrerer von einem zweiten Sprengmittel, Aromastoffe und Gleitmittel umfasst.

12. Sprühgetrocknete Formulierung nach Anspruch 11, wobei:
das Netzmittel ein Poloxamer, wie beispielsweise Poloxamer 188, Poloxamer 237, Poloxamer 338, Poloxamer 407 oder eine Kombination davon umfasst; oder
das Alkalisierungsmittel eine starke Base wie Kaliumhydroxid, Calciumhydroxid, Natriumhydroxid oder Bariumhydroxid ist; oder
der Puffer Dinatriumphosphat 2H₂O, Mononatriumphosphat, Zitronensäure/Dinatriumphosphat, Dinatriumphosphat-Hydrat, Bernsteinsäure/Natriumhydroxid, Zitronensäure/Natriumcitrat, Natriumcitrathydrat, Kaliumcitrat, Maleinsäure/Natriumhydroxid, Fumarsäure /Natriumhydroxid, Mononatriumphosphat-Hydrat, Monokaliumphosphat/Dinatriumphosphat, Dinatriumphosphat-Hydrat, Dikaliumphosphat, Dikaliumphosphat-Hydrat oder vorzugsweise Mononatriumphosphat, Mononatriumphosphat-Hydrat, Monokaliumphosphat/Dinatriumphosphat, Dinatriumphosphat-Hydrat, Dikaliumphosphat oder Dikaliumphosphat-Hydrat, oder bevorzugter Mononatriumphosphat, Mononatriumphosphat-Hydrat/Dinatriumphosphat oder Dinatriumphosphat-Hhydrat oder eine Kombination davon, vorzugsweise Dinatriumphosphat und Monophosphat ist; oder
der Süßstoff Sucralose, Aspartam, Acesulfam-Kalium, Saccharin, Steviolglykoside, Neotam, Advantam, Saccharin-Natrium, Cyclamat-Natrium oder Ammoniumglycyrrhizat oder eine Kombination davon ist; oder
das Bindemittel Hydroxypropylmethylcellulose (HPMC), wie HPMC 4 mPa.s oder HPMC 5 mPa.s, Hydroxypropylcellulose (HPC), Polyvinylpyrrolidon (PVP) oder Carboxymethylcellulose (CMC), Hydroxyethylmethylcellulose (HEMC), Hydroxyethylcellulose (HEC) oder eine Kombination davon ist; oder
das zweite Sprengmittel Natriumstärkeglykolat, Crospovidon, vorverkleisterte Stärke, Stärke oder Hydroxypropylcellulose (HPC) oder eine Kombination davon ist; oder
das Schmiermittel Polyethylenglykol, wie Polyethylenglykol 6000, Magnesiumstearat, Crospovidon, vorverkleisterte Stärke, Stärke oder Hydroxypropylcellulose (HPC) oder eine Kombination davon ist.

13. Sprühgetrocknete Formulierung nach einem der Ansprüche 8-12, wobei beim Kontakt mit der Zunge eines menschlichen Probanden keinen bitteren Geschmack hervorruft.

14. Sprühgetrocknete Formulierung, umfassend:
Fexofenadin-Zwitterion-Dihydrat;
ein Netzmittel;
ein Alkalisierungsmittel;
einen Puffer;
einen Süßstoff;
ein Bindemittel;
ein Verdünnungsmittel;
ein Sprengmittel;
einen Aromastoff; und
ein Schmiermittel.

15. Sprühgetrocknete Formulierung nach Anspruch 14, wobei:
das Netzmittel ein Poloxamer, wie Poloxamer 188, Poloxamer 407, Poloxamer 237, Poloxamer 338, ein Polysorbat, wie beispielsweise Polysorbat 80 oder eine Mischung aus Polysorbat 80 und Magnesiumaluminometasilikat, oder ein polyoxylhydriertes Rizinusöl, wie eine Mischung aus polyoxyl-40-hydriertem Rizinusöl und Magnesiumaluminosilikat, oder eine Kombination davon umfasst; oder
das Alkalisierungsmittel eine starke Base wie Kaliumhydroxid, Calciumhydroxid, Natriumhydroxid oder Bariumhydroxid ist; oder
der Puffer Dinatriumphosphat 2H₂O, Mononatriumphosphat, Zitronensäure/Dinatriumphosphat, Dinatriumphosphat-Hydrat, Bernsteinsäure/Natriumhydroxid, Zitronensäure/Natriumcitrat, Natriumcitrathydrat, Kaliumcitrat, Maleinsäure/Natriumhydroxid, Fumarsäure /Natriumhydroxid, Mononatriumphosphat-Hydrat, Monokaliumphosphat/Dinatriumphosphat, Dinatriumphosphat-Hydrat, Dikaliumphosphat, Dikaliumphosphat-Hydrat oder vorzugsweise Mononatriumphosphat, Mononatriumphosphat-Hydrat, Monokaliumphosphat/Dinatriumphosphat, Dinatriumphosphat-Hydrat, Dikaliumphosphat oder Dikaliumphosphat-Hydrat, oder bevorzugter Mononatriumphosphat, Mononatriumphosphat-Hydrat/Dinatriumphosphat oder Dinatriumphosphat-Hhydrat oder eine Kombination davon, vorzugsweise Dinatriumphosphat und Monophosphat ist; oder
der Süßstoff Sucralose, Aspartam, Acesulfam-Kalium, Saccharin, Steviolglycosid, Neotam, Advantam, Saccharin-Natrium, Cyclamat-Natrium oder Ammoniumglycyrrhizat oder eine Kombination davon ist; oder
das Bindemittel Hydroxypropylmethylcellulose (HPMC), wie HPMC 4 mPa.s oder HPMC 5 mPa.s, Hydroxypropylcellulose (HPC), Polyvinylpyrrolidon (PVP) oder Carboxymethylcellulose (CMC), Hydroxyethylmethylcellulose (HEMC), Hydroxyethylcellulose (HEC) oder eine Kombination davon ist; oder
das Verdünnungsmittel mikrokristalline Cellulose, Mannitol, Isomalt, Xylit, Maltitol, Maltodextrin, Lactose, Stärke, Sorbitol, Saccharose, ein Saccharosederivat, Invertzucker, lactitol, Erythrit, Fructose, Glucose, Calciumcarbonat, Cellulose, ein Cellulosederivat, Calciumcarbonat, Calciumlactat, Calciumsulfat oder Calciumphosphat oder eine Kombination davon, vorzugsweise mikrokristalline Cellulose und Mannitol ist; oder
das Sprengmittel Natriumcroscarmellose, Natriumstärkeglykolat, Natriumstärkeglykolat, Crospovidon, vorverkleisterte Stärke, Stärke oder Hydroxypropylcellulose (HPC) oder eine Kombination davon ist; oder
das Schmiermittel Polyethylenglykol, wie Polyethylenglykol 6000, Magnesiumstearat, Natriumstearylfumarat, Stearinsäure, Sorbitanmonostearat, Saccharosemonopalmitat, Glycerylmonostearat, Glyceryltribehenat, Glyceryldibehenat, Calciumstearat und Zinkstearat, hydratisiertes Magnesiumsilikat, Natriumoleat, essbares hydriertes Pflanzenöl, Natriumbenzoat, Talkum, Natriumacetat, ein Wachs, Natriumlaurylsulfat oder Magnesiumlaurylsulfat oder eine Kombination davon ist.

16. Formulierung nach Anspruch 14 oder 15, umfassend:
etwa 0,5 bis etwa 10 % w/w des Netzmittels; oder
etwa 0,5 bis etwa 5% w/w des Alkalisierungsmittels; oder
etwa 0,2 bis etwa 10 % w/w, des Puffers; oder
etwa 0,2 bis etwa 3% w/w des Süßstoffs; oder
etwa 0,5 bis etwa 5% w/w des Bindemittels; oder
etwa 30 bis etwa 80 % w/w des Verdünnungsmittels; oder
etwa 3 bis etwa 15 % w/w des Sprengmittels; oder
etwa 3 bis etwa 15 % w/w des Sprengmittels; oder
etwa 0,5 bis etwa 10 % w/w des Schmiermittels; oder
etwa 0,1 bis etwa 5% w/w des Aromastoffs.

17. Orale feste Darreichungsform, umfassend die sprühgetrocknete Formulierung nach einem der Ansprüche 14-16, optional ohne Überzugsschicht zur Maskierung bitterer Geschmacksstoffe.

18. Orale feste Darreichungsform nach Anspruch 17, die eine feste Darreichungsform, wie eine Tablette, z. *B.* eine Schmelztablette, eine Kautablette oder ein dispergierbares Granulat, oder eine flüssige Darreichungsform ist, wie eine Suspension, vorzugsweise eine Kautablette, die optional etwa 10 bis etwa 200 mg Fexofenadin-Zwitterion, beispielsweise etwa 30 bis etwa 180 mg, oder beispielsweise etwa 30 mg, etwa 60 mg, etwa 120 mg oder etwa 180 mg umfasst.

19. Sprühgetrocknete Formulierung nach einem der Ansprüche 8-16 oder orale feste Darreichungsform nach Anspruch 17 oder 18 zur Linderung von Symptomen aufgrund einer Allergie, wie einer Allergie der oberen Atemwege, bei einem Patienten, der diese benötigt.

20. Sprühgetrocknete Formulierung oder orale feste Darreichungsform nach Anspruch 19, wobei die Allergie auf ein Innenraumallergen wie Hausstaubmilben, ein Haustierallergen wie in Tierspeichel, Tierurin oder Tierhaaren oder Schimmelpilzen oder ein Außenallergen wie Pollen wie von Gräsern, Unkräutern oder Bäumen, wie Heuschnupfen oder Schimmelpilzen, zurückzuführen ist.

## Revendications

1. Procédé de préparation d'une formulation séchée par pulvérisation comprenant le zwittérion de la fexofénadine dihydrate comprenant le séchage par pulvérisation (i) d'une composition du zwittérion de la fexofénadine dihydrate ; et (ii) d'une matrice inerte.

2. Procédé selon la revendication 1, dans lequel la matrice inerte comprend un ou plusieurs excipients pharmaceutiquement acceptables tels qu'un diluant, un liant, un agent de glissement ou un désintégrant, ou une combinaison de ceux-ci.

3. Procédé selon la revendication 2, dans lequel
le diluant est de la cellulose microcristalline, du mannitol, du maltitol, du saccharose, de l'érythritol, du phosphate de calcium dibasique, du phosphate de calcium tribasique, de l'isomalt, du xylitol, de la maltodextrine, du lactose, de l'amidon, du sorbitol, du saccharose, un dérivé du saccharose, du saccharose inverti, de l'isomaltitol, de l'érythritol, du fructose, du glucose, du carbonate de calcium, de la cellulose, un dérivé de la cellulose, du carbonate de calcium, du lactate de calcium, du sulfate de calcium ou du phosphate de calcium, ou une combinaison de ceux-ci ; ou
le désintégrant est la croscarmellose de sodium, du glycolate d'amidon sodique, la crospovidone, l'amidon prégélatinisé, l'amidon ou l'hydroxypropylcellulose (HPC), ou une combinaison de ceux-ci.

4. Procédé selon l'une quelconque des revendications 1-3, comprenant en outre :
(a) la combinaison du chlorhydrate de fexofénadine, de l'eau et d'un agent mouillant;
(b) la combinaison d'un agent alcalinisant avec le produit de l'étape (a) ;
(c) l'ajustement du pH du produit de l'étape (b) à un pH de 5,8 à 7 en utilisant un ou plusieurs tampons;
d) la combinaison d'un édulcorant avec le produit de l'étape (c) ; et
(e) la combinaison du produit de l'étape (d) avec un liant ;
dans lequel la composition du zwittérion de la fexofénadine dihydrate présente un pH de 6,5 à 7, de préférence de 6,7.

5. Procédé selon la revendication 4, comprenant en outre la combinaison de la formulation séchée par pulvérisation avec un ou plusieurs d'une seconde portion de désintégrant, d'un second désintégrant, d'une seconde portion de diluant, d'un agent aromatisant, d'une seconde portion d'agent mouillant et d'un ou de plusieurs lubrifiants.

6. Procédé selon la revendication 5, dans lequel :
le second désintégrant est le glycolate d'amidon sodique, la crospovidone, l'amidon prégélatinisé, l'amidon, l'hydroxypropylcellulose (HPC) ou une combinaison de ceux-ci ; ou
le lubrifiant est du polyéthylène glycol (PEG) tel que le PEG 6000, du stéarate de magnésium, du fumarate de stéaryle de sodium, de l'acide stéarique, du monostéarate de sorbitan, du monopalmitate de saccharose, du monostéarate de glycéryle, du tribéhénate de glycéryle, du dibéhénate de glycéryle, du stéarate de calcium et du stéarate de zinc, du silicate de magnésium hydraté, de l'oléate de sodium, de l'huile végétale hydrogénée comestible, du benzoate de sodium, du talc, de l'acétate de sodium, une cire, du laurylsulfate de sodium ou du laurylsulfate de magnésium, ou une combinaison de ceux-ci.

7. Produit préparé selon le procédé décrit dans l'une quelconque des revendications précédentes.

8. Formulation séchée par pulvérisation comprenant le zwittérion de la fexofénadine dihydrate et une matrice inerte.

9. Formulation séchée par pulvérisation selon la revendication 8, dans laquelle la matrice inerte comprend un ou plusieurs excipients pharmaceutiquement acceptables, tels qu'un diluant ou un premier désintégrant.

10. Formulation séchée par pulvérisation selon la revendication 9, dans laquelle :
le diluant est de la cellulose microcristalline, du mannitol, de l'isomalt, du xylitol ou une combinaison de ceux-ci, de préférence de la cellulose microcristalline et du mannitol, de préférence de la cellulose microcristalline, du mannitol, du maltitol, de la maltodextrine, du lactose, de l'amidon, du sorbitol, du saccharose, un dérivé du saccharose, du saccharose inverti, de l'isomaltitol, de l'érythritol, du fructose, du glucose, du carbonate de calcium, de la cellulose, un dérivé de la cellulose, du carbonate de calcium, du lactate de calcium, du sulfate de calcium ou du phosphate de calcium, ou une combinaison de ceux-ci ; ou
le premier désintégrant est la croscarmellose sodique, le glycolate d'amidon sodique, la crospovidone, l'amidon prégélatinisé, l'amidon, l'hydroxypropylcellulose (HPC) ou une combinaison de ceux-ci.

11. Formulation séchée par pulvérisation selon l'une quelconque des revendications 8-10, dans laquelle la formulation avant séchage par pulvérisation comprend en outre un ou plusieurs d'un agent mouillant, agent alcalinisant, tampon, édulcorant, liant ou solvant, comprenant optionnellement en outre un ou plusieurs d'un second désintégrant, agent aromatisant et lubrifiant.

12. Formulation séchée par pulvérisation selon la revendication 11, dans laquelle :
l'agent mouillant comprend un poloxamère, tel que le poloxamère 188, le poloxamère 237, le poloxamère 338, le poloxamère 407, ou une combinaison de ceux-ci ; ou
l'agent alcalinisant est une base forte telle que l'hydroxyde de potassium, l'hydroxyde de calcium, l'hydroxyde de sodium ou l'hydroxyde de baryum ; ou
le tampon est le phosphate disodique (2H₂O), le phosphate monosodique (2H₂O), l'acide citrique/phosphate dibasique, le phosphate disodique hydraté, l'acide succinique /hydroxyde de sodium, l'acide citrique /le citrate de sodium, le citrate de sodium hydraté, le citrate de potassium, l'acide maléique /hydroxyde de sodium, l'acide fumarique /hydroxyde de sodium, le phosphate monosodique hydraté, le phosphate de potassium monobasique/phosphate de sodium dibasique, le phosphate disodique hydraté, le phosphate dipotassique, le phosphate disodique hydraté, ou de préférence le phosphate monosodique, le phosphate monosodique hydraté, le phosphate de potassium monobasique/phosphate de sodium dibasique, le phosphate disodique hydraté, le phosphate dibasique de potassium, ou le phosphate disodique hydraté de potassium, ou de préférence le phosphate monosodique, le phosphate monosodique hydraté/phosphate de sodium dibasique, ou le phosphate disodique hydraté, ou une combinaison de ceux-ci, ou de préférence le phosphate dibasique et le phosphate monobasique ; ou
l'édulcorant est du sucralose, de l'aspartame, de l'acésulfame potassium, de la saccharine, des glycosides de stéviol, du néotame, de l'advantame, de la saccharine sodique, du cyclamate de sodium ou du glycyrrhizate d'ammonium, ou une combinaison de ceux-ci ; ou
le liant est l'hydroxypropylméthylcellulose (HPMC), telle que l'HPMC 4mPa. s ou l'HPMC 5 mPa.s., l'hydroxypropylcellulose (HPC), la polyvinylpyrrolidone (PVP), ou la carboxyméthylcellulose (CMC), l'hydroxyéthylméthylcellulose (HEMC), ou l'hydroxyéthylcellulose (HEC), ou une combinaison de ceux-ci ; ou
le second désintégrant est le glycolate d'amidon sodique, la crospovidone, l'amidon prégélatinisé, l'amidon ou l'hydroxypropylcellulose (HPC), ou une combinaison de ceux-ci ; ou
le lubrifiant est du polyéthylène glycol, tel que le polyéthylène glycol 6000, du stéarate de magnésium, de la crospovidone, de l'amidon prégélatinisé, de l'amidon ou de l'hydroxypropylcellulose (HPC), ou une combinaison de ceux-ci.

13. Formulation séchée par pulvérisation selon l'une quelconque des revendications 8-12, dans laquelle, au contact de la langue d'un sujet humain, ne produit pas de goût amer.

14. Formulation séchée par pulvérisation, comprenant :
le zwittérion de la fexofénadine dihydrate ;
un agent mouillant;
un agent alcalinisant ;
un tampon ;
un édulcorant ;
un liant ;
un diluant ;
un désintégrant ;
un agent aromatisant ; et
un lubrifiant.

15. Formulation séchée par pulvérisation selon la revendication 14, dans laquelle :
l'agent mouillant comprend un poloxamère, tel que le poloxamère 188, le poloxamère 407, le poloxamère 237, le poloxamère 338, un polysorbate tel que le polysorbate 80 ou un mélange de polysorbate 80 et d'aluminométasilicate de magnésium, ou une huile de ricin hydrogénée polyoxylée, telle qu'un mélange d'huile de ricin hydrogénée polyoxylée 40 et d'aluminosilicate de magnésium, ou une combinaison de ceux-ci ; ou
l'agent alcalinisant est une base forte telle que l'hydroxyde de potassium, l'hydroxyde de calcium, l'hydroxyde de sodium ou l'hydroxyde de baryum ; ou
le tampon est le phosphate disodique dihydraté (2H₂O), le phosphate monosodique (2H₂O), l'acide citrique, le phosphate de sodium dibasique, le phosphate disodique hydraté, l'acide succinique /hydroxyde de sodium, l'acide citrique /citrate de sodium, le citrate de sodium hydraté, le citrate de potassium, l'acide maléique /hydroxyde de sodium, l'acide fumarique/hydroxyde de sodium, le phosphate monosodique hydraté, le phosphate de potassium monobasique/le phosphate de sodium dibasique, le phosphate disodique hydraté, le phosphate dipotassique, le phosphate disodique hydraté, ou de préférence le phosphate monosodique, le phosphate monosodique hydraté, le phosphate de potassium monobasique/le phosphate de sodium dibasique, le phosphate disodique hydraté, le phosphate dibasique de potassium, ou le phosphate disodique hydraté de potassium, ou, plus préférentiellement, le phosphate monosodique, le phosphate monosodique hydrate/le phosphate de sodium dibasique, ou le phosphate disodique hydraté, ou une combinaison de ceux-ci, ou de préférence le phosphate dibasique et le phosphate monobasique ; ou
l'édulcorant est le sucralose, l'aspartame, l'acésulfame potassium, la saccharine, le glycoside de stéviol, le néotame, l'advantame, la saccharine sodique, le cyclamate de sodium ou le glycyrrhizate d'ammonium, ou une combinaison de ceux-ci ; ou
le liant est l'hydroxypropylméthylcellulose (HPMC), telle que l'HPMC 4 mPa. s ou l'HPMC 5 mPa.s, l'hydroxypropylcellulose (HPC), la polyvinylpyrrolidone (PVP), ou la carboxyméthylcellulose (CMC), l'hydroxyéthylméthylcellulose (HEMC), ou l'hydroxyéthylcellulose (HEC), ou une combinaison de ceux-ci ; ou
le diluant est de la cellulose microcristalline, du mannitol, de l'isomalt, du xylitol, du maltitol, de la maltodextrine, du lactose, de l'amidon, du sorbitol, du saccharose, un dérivé du saccharose, du saccharose inverti, de l'isomaltitol, de l'érythritol, du fructose, du glucose, du carbonate de calcium, de la cellulose, un dérivé de la cellulose, du carbonate de calcium, du lactate de calcium, du sulfate de calcium ou du phosphate de calcium, ou une combinaison de ceux-ci, de préférence de la cellulose microcristalline et du mannitol ; ou
le désintégrant est la croscarmellose sodique, le glycolate d'amidon sodique, la crospovidone, l'amidon prégélatinisé, l'amidon ou l'hydroxypropylcellulose (HPC), ou une combinaison de ceux-ci ; ou
le lubrifiant est du polyéthylène glycol, tel que le polyéthylène glycol 6000, le stéarate de magnésium, le fumarate de stéaryle de sodium, l'acide stéarique, le monostéarate de sorbitan, le monopalmitate de saccharose, le monostéarate de glycéryle, le tribéhénate de glycéryle, le dibéhénate de glycéryle, le stéarate de calcium et le stéarate de zinc, le silicate de magnésium hydraté, l'oléate de sodium, l'huile végétale hydrogénée comestible, le benzoate de sodium, de talc, l'acétate de sodium, une cire, le laurylsulfate de sodium ou le laurylsulfate de magnésium, ou une combinaison de ceux-ci.

16. Formulation séchée par pulvérisation selon la revendication 14 ou 15, comprenant :
environ 0,5 à environ 10% p/p de l'agent mouillant ; ou
environ 0,5 à environ 5% p/p de l'agent alcalinisant ; ou
environ 0,2 à environ 10% p/p, de la zone tampon ; ou
environ 0,2 à environ 3% p/p de l'édulcorant ; ou
environ 0,5 à environ 5% p/p du liant ; ou
environ 30 à environ 80 % p/p du diluant ; ou
environ 3 à environ 15% p/p du désintégrant ; ou
environ 3 à environ 15% p/p du désintégrant ; ou
environ 0,5 à environ 10% p/p du lubrifiant ; ou
environ 0,1 à environ 5% p/p de l'agent aromatisant.

17. Forme posologique orale solide comprenant la formulation séchée par pulvérisation selon l'une quelconque des revendications 14-16, optionnellement dépourvue d'une couche d'enrobage pour masquer le goût amer.

18. Forme posologique solide orale selon la revendication 17, c'est-à-dire une forme posologique solide, telle qu'un comprimé, *par exemple* un comprimé à désintégration orale, un comprimé à mâcher ou des granules dispersibles, ou une forme posologique liquide, telle qu'une suspension, de préférence un comprimé à mâcher, comprenant optionnellement environ 10 à environ 200 mg du zwittérion de la fexofénadine, par exemple environ 30 à environ 180 mg, ou par exemple environ 30 mg, environ 60 mg, environ 120 mg ou environ 180 mg.

19. Formulation séchée par pulvérisation selon l'une quelconque des revendications 8-16 ou forme posologique solide orale de la revendication 17 ou 18 pour soulager les symptômes dus à une allergie telle qu'une allergie des voies respiratoires supérieures chez un patient qui en a besoin.

20. Formulation séchée par pulvérisation ou forme posologique solide orale selon la revendication 19, dans laquelle l'allergie est due à un allergène d'intérieur tel que les acariens de la poussière, un allergène d'animal de compagnie tel que la salive animale, l'urine animale ou les pellicules animales, ou la moisissure ou un allergène d'extérieur tel que le pollen tel que celui de l'herbe, des mauvaises herbes ou des arbres, comme le rhume des foins ou la moisissure.
